# EUROPEAN PATENT APPLICATION

(11) **EP 4 578 861 A1**
(43) Date of publication of application: **02.07.2025**
(21) Application number: 23873219.2
(22) Date of filing: 27.09.2023
(51) Int. Cl.: C07D 493/04, A61K 31/5377, A61K 31/496, A61K 31/4025, A61K 31/453, A61P 35/00

(54) **NOVEL TRICYCLIC HETEROCYCLIC CARBALDEHYDE COMPOUND AND PHARMACEUTICAL COMPOSITION, CONTAINING SAME, FOR IRE1ALPHA INHIBITION**

(30) Priority: 30.09.2022 KR 20220125743
(71) Applicant: Hanmi Pharm. Co., Ltd., Hwaseong-si, Gyeonggi-do 18536 (KR)
(72) Inventor: KIM, Ji Sook, Hwaseong-si, Gyeonggi-do 18469 (KR); JUNG, Seung Hyun, Hwaseong-si, Gyeonggi-do 18469 (KR); KIM, Min Jeong, Hwaseong-si, Gyeonggi-do 18469 (KR); PARK, Won Gi, Hwaseong-si, Gyeonggi-do 18469 (KR); CHOI, Jae Yul, Hwaseong-si, Gyeonggi-do 18469 (KR); AHN, Young Gil, Hwaseong-si, Gyeonggi-do 18469 (KR)
(74) Representative: Brevalex
(86) International application number: PCT/KR2023/015052
(87) International publication number: WO 2024/072110

(57) **Abstract**

The present disclosure relates to a novel tricyclic heterocyclic carbaldehyde compound and a pharmaceutical composition for inhibiting IRE1α including the same. More specifically, the present disclosure relates to a novel tricyclic heterocyclic carbaldehyde compound for treating or preventing IRE1α-associated diseases and a pharmaceutical composition including the same.

## Description

### Technical Field

The present disclosure relates to a novel tricyclic heterocyclic carbaldehyde compound and a pharmaceutical composition for inhibiting inositol requiring enzyme 1α (IRE1α) including the same, and more particularly, to a novel tricyclic heterocyclic carbaldehyde compound for treating or preventing IRE1α-associated diseases and a pharmaceutical composition including the same.

### Background Art

A delicate balance exists between cell survival and death depending on how protein folding stress is managed by cells. Imbalances in protein homeostasis cause many metabolic, tumor, neurodegenerative, inflammatory, cardiovascular, and infectious diseases.

Cancer cells use the unfolded protein response (UPR) to relieve endoplasmic reticulum stress (ER stress) caused by cellular oncogene activation and the hostile tumor microenvironment (TME) (Non-Patent Document 1). The UPR is activated in many human cancers and is important for tumor initiation, tumor progression, and tumor treatment resistance. Endoplasmic reticulum stress due to unfolded proteins in a cell's endoplasmic reticulum initiates a signaling cascade of the UPR. Inositol requiring enzyme 1α (IRE1α) is an endoRNase present in the endoplasmic reticulum membrane. Under stress-free conditions, IRE1α binds to binding immunoglobulin protein (BIP) and remains in an inactive state, but when endoplasmic reticulum stress occurs, it is phosphorylated and activated by the detachment of bound BIP. The activated IRE1α splices the mRNA of a transcription factor called X-box binding protein 1 (XBP1) to induce an activated XBP-1 form, and the activated XBP1 binds to the ER stress response element and induces transcriptional activity. Thus, inositol requiring enzyme 1α (IRE1α), a key enzyme of the UPR, relieves protein folding stress and protects cells from stress-induced apoptosis.

The IRE1α gene is frequently amplified and overexpressed in aggressive luminal B breast cancer cells, which is significantly associated with poor prognosis of breast cancer patients (Non-Patent Document 2). In addition, triple-negative breast cancer (TNBC) is a very aggressive malignant tumor with a poor prognosis and limited treatment options due to the lack of effective targeted therapies to date. XBP1 is activated in TNBC and plays a pivotal role in tumorigenesis and progression of breast cancer subtypes (Non-Patent Document 3). Oncogenic MYC is known to regulate the IRE1α/XBP1 pathway of the UPR in breast cancer through multiple mechanisms. In an anticancer efficacy test on IRE1α or XBP1 knockout genetically engineered MYC amplified TNBC mouse models, MYC-overexpressing tumor growth is selectively inhibited (Non-Patent Document 4). Meanwhile, research is underway (Patent Documents 1 and 2) to develop substances that inhibit IRE1α to treat diseases associated with unfolded protein responses, but the research is limited.

The inventors of the present application developed a novel tricyclic heterocyclic carbaldehyde compound capable of inhibiting IRE1α, thereby completing the present disclosure.

### [Prior art documents]

### [Patent documents]

(Patent Document 1) International Publication Patent WO2011/127070
(Patent Document 2) International Publication Patent WO2011/056744

### Non-Patent Documents

(Non-Patent Document 1) Cancer Res. 2020, 80(11), 2368
(Non-Patent Document 2) iScience. 2020, 23(9), 101503
(Non-Patent Document 3) Nature 2014, 508 (7494), 103
(Non-Patent Document 4) Cancer Res 2020, 80(11), 2368.

### Disclosure

### Technical Problem

An objective of the present disclosure is to provide a novel tricyclic heterocyclic carbaldehyde compound having excellent inhibitory activity against inositol requiring enzyme 1α (IRE1α).

Another objective of the present disclosure is to provide a pharmaceutical composition for treating or preventing IRE1α-associated diseases including the compound as an active ingredient.

Other objectives and advantages of this application will become more apparent from the following detailed description taken in conjunction with the appended claims. Matters not set forth in this specification are sufficiently obvious to be recognized and inferred by those skilled in the art of the present application or technical fields similar thereto, so the description thereof will be omitted.

### Technical Solution

An embodiment of the present disclosure provides a compound selected from compounds of Formula 1, optical isomers, diastereomers, solvates, and hydrates thereof, and pharmaceutically acceptable salts thereof.

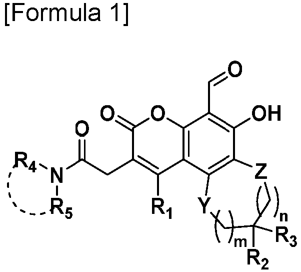

Another embodiment of the present disclosure provides a pharmaceutical composition for treating or preventing IRE1α-associated diseases including, as an active ingredient, a compound selected from compounds of Formula 1, optical isomers, diastereomers, solvates, and hydrates thereof, and pharmaceutically acceptable salts thereof.

### Advantageous Effects of Disclosure

The tricyclic heterocyclic carbaldehyde compound having the structure of Formula 1 according to an embodiment of the present disclosure and the pharmaceutical composition including the same, are effective in inhibiting IRE1α protein and may be useful as a therapeutic agent therefor.

### Mode for Invention

Hereinafter, the present disclosure will be described in more detail.

All technical terms used in the present disclosure, unless defined otherwise, are used with the same meaning as commonly understood by those skilled in the art associated to the present disclosure. In addition, although preferred methods or samples are described in this specification, those similar or equivalent thereto are also included in the scope of the present disclosure.

An embodiment of the present disclosure provides a compound selected from compounds of Formula 1, optical isomers, diastereomers, solvates, and hydrates thereof, and pharmaceutically acceptable salts thereof. 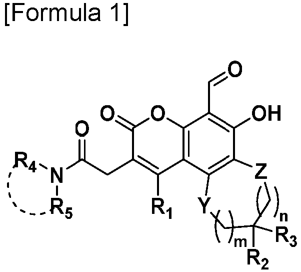 wherein, in Formula 1,
R₁ may be hydrogen, halogen, hydroxy, cyano, C₁₋₆ alkyl, C₂₋₆ alkenyl, C₂₋₆ alkynyl, haloC₁₋₆ alkyl, -(CH₂)ₐ-C₁₋₆ alkoxy, -(CH₂)ₐ-NR₆R₇ substituted or unsubstituted - (CH₂)ₐ-C₃₋₆ cyclyl, substituted or unsubstituted -(CH₂)ₐ-C₆₋₁₀ aryl, or -(CH₂)ₐ-C₂₋₆ heterocyclyl;
R₂ and R₃ may each independently be hydrogen, halogen, C₁₋₆ alkyl, haloC₁₋₆ alkyl, C₁₆ alkoxy, oxo(=O), or hydroxy;
a may be an integer from 0 to 2;
n and m may each independently be an integer of 0 to 3;
Z and Y may each independently be -CH₂-, -O-, -S-, or -NR₈-;
the dotted line indicates the presence or absence of bonding;
R₄ and R₅ may each independently be hydrogen, C₁₋₆ alkyl, C₂₋₆ alkenyl, C₂₋₆ alkynyl, C₁₋₆ alkoxy, C₁₋₆ alkoxyalkyl, C₃₋₁₀ cycloalkyl, C₆₋₁₀ aryl, C₄₋₁₀ heteroaryl, C₂₋₉ heterocycloalkyl, C₈₋₁₆ spirocycloalkyl, C₈₋₁₆ fused cycloalkyl, C₈₋₁₆ cross-linked cycloalkyl, C₆₋₁₄ heterospirocycloalkyl, C₆₋₁₄ fused heterocycloalkyl, or C₆₋₁₄ cross-linked heterocycloalkyl, each of which may be unsubstituted or substituted with at least one substituent selected from halogen, nitro, oxo(=O), cyano, haloC₁₋₆ alkyl, C₁₋₆ alkyl, C₂₋₆ alkenyl, C₂₋₆ alkynyl, C₁₋₆ alkoxy, haloC₁₋₆ alkoxy, -S(O)-C₁₋₄ alkyl, -S(O)₂-C₁₋₄ alkyl, - C(O)-NR₉R₁₀, -C(O)OR₉, -OR₉, and -NR₉R₁₀;
alternatively, R₄ and R₅ may be linked to each other to form, with the amide nitrogen of Formula 1, C₂₋₉ heterocyclyl, C₆₋₁₄ heterospirocyclyl, C₆₋₁₄ fused heterocyclyl, C₆₋₁₄ cross-linked heterocyclyl, or C₄₋₁₀ heteroaryl, each of which may be unsubstituted or substituted with one or more substituents selected from halogen, nitro, oxo(=O), cyano, haloC₁₋₆ alkyl, C₁₋₆ alkyl, C₂₋₆ alkenyl, C₂₋₆ alkynyl, C₁₋₆ alkoxy, haloC₁₋₆ alkoxy, - C(O)R₁₁, -S(O)-C₁₋₄ alkyl, -S(O)₂-C₁₋₄ alkyl, -C(O)-NR₁₁R₁₂, -C(O)OR₁₁, -OR₁₁, and - NR₁₁R₁₂;
R₆, R₇, R₈, R₉, R₁₀, R₁₁, and R₁₂ may each independently be hydrogen, C₁₋₆ alkyl, C₂₋₆ alkenyl, C₂₋₆ alkynyl, C₁₋₆ alkoxy, C₁₋₆ alkoxyalkyl, haloC₁₋₆ alkyl, C₆₋₁₀ aryl, C₃₋₁₀ cycloalkyl, C₄₋₁₀ heteroaryl, or C₂₋₉ heterocycloalkyl, alternatively, when R₁ is -(CH₂)ₐ-NR₆R₇, or when R₄ and R₅ are C(O)-NR₉R₁₀ or -NR₉R₁₀, a pair of R₆ and R₇, or a pair of R₉ and R₁₀ may be linked to each other to form, with an amide nitrogen, a substituted or unsubstituted C₂₋₉ heterocyclyl or a substituted or unsubstituted C₄₋₁₀ heteroaryl.

The term "halogen" herein may be F, Cl, Br, or I.

The term "alkyl" as used herein refers to, unless specified otherwise, a linear or branched hydrocarbon moiety which may be substituted or unsubstituted. The alkyl group may be, for example, methyl, ethyl, propyl, butyl, pentyl, isopropyl, isobutyl, or t-butyl, but is not limited thereto.

The term "alkenyl" as used herein refers to, unless specified otherwise, an alkyl group including one or more double bonds, which may be substituted or unsubstituted. The alkenyl group may be, for example, prop-1-ene, but-1-ene, but-2-ene, 3-methylbut-1-ene, or pent-1-ene, but is not limited thereto.

The term "alkynyl" as used herein refers to, unless specified otherwise, an alkyl group including one or more triple bonds, which may be substituted or unsubstituted. The alkynyl group may be, for example, ethynyl, propynyl, butynyl, pentynyl, hexynyl, or a branched chain alkynyl group, but is not limited thereto.

The term "cycloalkyl" as used herein refers to, unless specified otherwise, a saturated monocyclic or polycyclic hydrocarbon ring generally having the specified number of carbon atoms, the saturated monocyclic or polycyclic hydrocarbon ring being substituted or unsubstituted. The cycloalkyl group may be, for example, cyclopropyl, cyclobutyl, cyclopentyl, cyclohexyl, or cycloheptyl, but is not limited thereto.

The term "heterocycloalkyl" as used herein refers to, unless specified otherwise, a monocyclic alkyl including one or more heteroatoms selected from B, N, O, S, P(=O), Si, and P, being substituted or unsubstituted. The heterocycloalkyl group may be, for example, piperidinyl, piperazinyl, morpholinyl, pyrrolidinyl, thiomorpholinyl, imidazolidinyl, tetrahydrofuryl, or groups similar thereto, but are not limited thereto.

The term "spiro" as used herein refers to, unless specified otherwise, two rings that share one atom, and refers to the case where the two rings are not linked to each other by cross-linking (bridge). The term "spiro linkage" as used herein refers to, unless specified otherwise, a linking group that shares one atom.

The term "spirocycloalkyl" as used herein refers to, unless specified otherwise, a saturated carbocyclic compound including two rings, in which both rings share only one carbon atom as part of the rings. The spirocycloalkyl may be, for example, 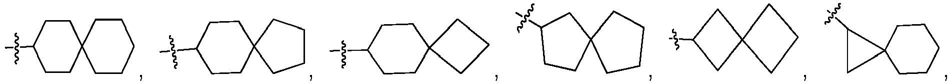 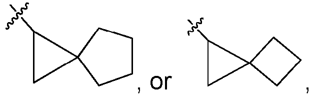 but is not limited thereto.

The term "heterospirocycloalkyl" as used herein refers to, unless specified otherwise, a spirocycloalkyl including one or more heteroatoms selected from B, N, O, S, P(=O), Si, and P. The heterospirocycloalkyl may be, for example, 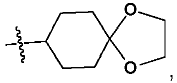 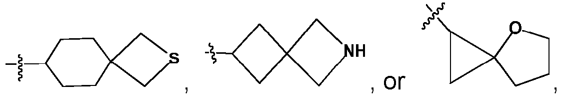 but is not limited thereto.

The term "cross-linked cycloalkyl" as used herein refers to, unless specified otherwise, two rings that share two common non-adjacent ring atoms. Cross-linked cycloalkyls may be classified as bicyclic, tricyclic, tetracyclic or polycyclic cross-linked cycloalkyls, according to the number of rings. The cross-linked cycloalkyl may be, for example, 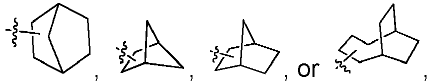 but is not limited thereto.

The term "cross-linked heterocycloalkyl" as used herein refers to, unless specified otherwise, a cross-linked cycloalkyl including one or more heteroatoms selected from B, N, O, S, P(=O), Si, and P.

The term "fused cycloalkyl" as used herein refers to, unless specified otherwise, a group in which each ring shares a pair of carbon atoms adjacent to a different ring, and one or more rings may share one or more double bonds, but neither of these rings does not have a complete conjugated π-electron system. Fused cycloalkyls may be classified as bicyclic, tricyclic, tetracyclic or polycyclic fused cycloalkyls, according to the number of rings. The fused cycloalkyl may be, for example, 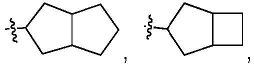 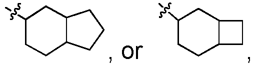 but is not limited thereto.

The term "fused heterocycloalkyl" as used herein refers to, unless specified otherwise, a fused cycloalkyl including one or more heteroatoms selected from B, N, O, S, P(=O), Si, and P.

The term "haloalkyl" as used herein includes monohaloalkyl and polyhaloalkyl, which may be substituted or unsubstituted, unless specified otherwise. The terms "halogen" and "alkyl" are the same as defined above.

The term "alkoxy" as used herein refers to, unless specified otherwise, a group in which a linear or branched hydrocarbon moiety is linked to oxygen, the group being substituted or unsubstituted. The alkoxy may be, for example, methoxy, ethoxy, propoxy, and butoxy, or isopropoxy, isobutoxy, or t-butoxy, but is not limited thereto.

The term "alkoxyalkyl" as used herein refers to, unless specified otherwise, a substituted or unsubstituted group in which that a linear or branched alkyl group is substituted with an alkoxy group. The alkoxyalkyl group may be, for example, a methoxymethyl group, an ethoxymethyl group, a propoxymethyl group, an isopropoxymethyl group, a methoxyethyl group, an ethoxyethyl group, or a methoxypropyl group, but is not limited thereto.

The term "aryl" as used herein refers to, unless specified otherwise, an aromatic group that may be substituted or unsubstituted, and may include, for example, C₃-C₁₀ aryl, C₃-C₈ aryl, or C₃-C₆ aryl, in which a double bond alternates (resonates) between adjacent carbon atoms or suitable heteroatoms. For example, the aryl may be phenyl, biphenyl, naphthyl, toluyl, or naphthalenyl, but is not limited thereto.

The term "heteroaryl" as used herein refers to, unless specified otherwise, a monocyclic or bicyclic or more, substituted or unsubstituted, aromatic group including one or more heteroatoms selected from B, N, O, S, P(=O), Si, and P.

The term "heterocyclyl" as used herein refers to, unless specified otherwise, a monocyclic hydrocarbon including one or more heteroatoms selected from B, N, O, S, P(=O), Si, and P, being substituted or unsubstituted. The term "heterocycloalkyl" as used herein is an example of "heterocyclyl." The heterocyclyl may be, for example, substituted or unsubstituted, 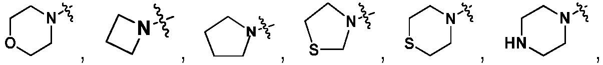 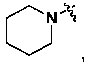 or a group similar thereto, but is not limited thereto.

The term "heterospirocyclyl" as used herein refers to, unless specified otherwise, a saturated or carbocyclic compound including two rings in which both rings share only one carbon atom as part of the rings. The term "heterospirocycloalkyl" as used herein is an example of "heterospirocyclyl." The heterospirocyclyl may be, for example, 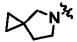 or a group similar thereto, but is not limited thereto.

The term "fused heterocyclyl" as used herein refers to, unless specified otherwise, a group that includes one or more heteroatoms selected from B, N, O, S, P(=O), Si, and P, in which each ring shares a pair of carbon atoms adjacent to a different ring, and one or more rings may share one or more double bonds, but none of these rings have a complete conjugated π-electron system, and may be catagorized as bicyclic, tricyclic, tetracyclic or polycyclic fused heterocyclyls, depending on the number of rings. The term "fused heterocycloalkyl" as used herein is an example of the term "fused heterocyclyl."

The term "cross-linked heterocyclyl" as used herein refers to, unless specified otherwise, two or more rings which include one or more heteroatoms selected from B, N, O, S, P(=O), Si, and P, may be saturated or unsaturated, and share two non-adjacent common ring atoms, and may be catagorized as bicyclic, tricyclic, tetracyclic or polycyclic cross-linked heterocyclyls, depending on the number of rings. The cross-linked heterocyclyl may be, for example, 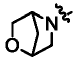 and the like, but is not limited thereto. The term "cross-linked heterocycloalkyl" may be an example of the term "cross-linked heterocyclyl."

The term "stereoisomers" as used herein refers to compounds of the present disclosure or salts thereof which have the same chemical formula or molecular formula and are optically or sterically different, and includes optical isomers or diastereomers.

The term "optical isomers" as used herein refers to two stereoisomers of a compound that are non-superimposable mirror images of each other.

The term "diastereomer" as used herein refers to a stereoisomer having two or more chiral centers, the molecules of which are not mirror images of each other.

Compounds of the present disclosure may include asymmetric or chiral centers and thus may exist in different stereoisomeric forms. All stereoisomeric forms of the compounds of the present disclosure, such as diastereomers, optical isomers and racemic mixtures, are considered to form part of the present disclosure. A 50:50 mixture of optical isomers is called a racemic mixture or racemate.

The term "solvate" as used herein refers to a compound of the present disclosure or a salt thereof, including a stoichiometric or non-stoichiometric amount of a solvent bound by non-covalent intermolecular forces. Preferred solvents in this regard may be solvents that are volatile, non-toxic, and/or suitable for administration to humans. The "solvate" may include a molecular complex comprising the compound and one or more pharmaceutically acceptable solvent molecules, such as ethanol.

The term "hydrate" as used herein refers to a complex in which the solvent molecule is water.

The term "pharmaceutically acceptable salt" as used herein refers to a pharmaceutically acceptable organic or inorganic salt, which may be prepared by any suitable method useful to those skilled in the art. For example, when a compound of the present disclosure is a base, the pharmaceutically acceptable salt may be prepared by any suitable method useful to those skilled in the art, for example, by treating the free base with an inorganic or organic acid or the like.

In an embodiment, R₁ may be C₁₋₆ alkyl, haloC₁₋₆ alkyl, -(CH₂)ₐ-C₁₋₆ alkoxy, - (CH₂)ₐ-NR₆R₇, or substituted or unsubstituted -(CH₂)ₐ-C₃₋₆ cyclyl.

In an embodiment, R₂ and R₃ may each independently be hydrogen, halogen, or C₁₋₆ alkyl.

In an embodiment, Z and Y may each independently be -CH₂- or -O-.

In an embodiment, R₄ and R₅ may each independently be: hydrogen, C₁₋₆ alkyl, C₂₋₆ alkenyl, C₂₋₆ alkynyl, haloC₁₋₆ alkyl, C₁₋₆ alkoxyalkyl, C₃₋₁₀ cycloalkyl, or C₂₋₉ heterocycloalkyl; or C₁₋₆ alkyl substituted with C₂₋₉ heterocycloalkyl.

In an embodiment, R₄ and R₅ may each independently be hydrogen, methyl, ethyl, propyl, methoxymethyl, methoxyethyl, ethoxymethyl, ethoxyethyl, morpholinomethyl, or morpholinoethyl.

In an embodiment, R₄ and R₅ may be linked to each other, with the amide nitrogen of Formula 1, to form C₂₋₉ heterocyclyl, C₆₋₁₄ heterospirocyclyl, C₆₋₁₄ cross-linked heterocyclyl, or C₄₋₁₀ heteroaryl, which is one of 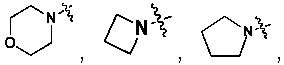 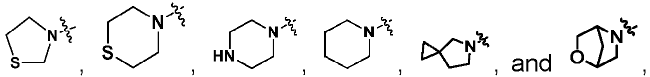 wherein C₂₋₉ heterocyclyl, C₆₋₁₄ heterospirocyclyl, C₆₋₁₄ cross-linked heterocyclyl, or C₄₋₁₀ heteroaryl may be substituted with at least one substituent selected from hydrogen, halogen, nitro, cyano, haloC₁₋₆ alkyl, C₁₋₆ alkyl, C₂₋₆ alkenyl, C₂₋₆ alkynyl, C₁₋₆ alkoxy, -C(O)R₁₁, haloC₁₋₆ alkoxy, and C₂₋₆ heterocyclyl.

In an embodiment, R₄ and R₅ may be linked to each other to form, with the amide nitrogen of Formula 1, one selected from 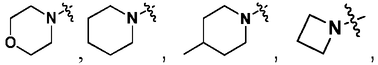 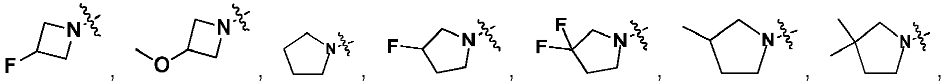 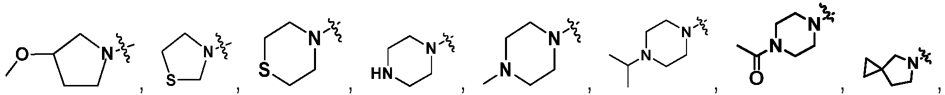 and

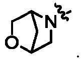

In an embodiment,
R₁ may be C₁₋₆ alkyl,
R₂ and R₃ may each independently be hydrogen, halogen, C₁₋₆ alkyl, haloC₁₋₆ alkyl, C₁₋₆ alkoxy, oxo(=O), or hydroxy;
n and m may each independently be an integer from 0 or 1;
Z and Y may each independently be -CH₂- or -O-;
R₄ and R₅ may be linked to each other to form, with the amide nitrogen of Formula 1, a C₂₋₉ heterocyclyl, C₆₋₁₄ heterospirocyclyl, C₆₋₁₄ cross-linked heterocyclyl, or C₄₋₁₀ heteroaryl, which is one of 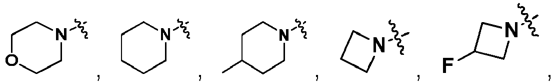 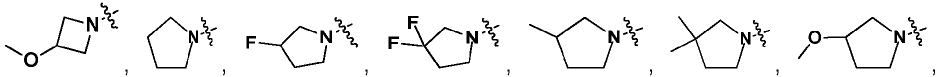 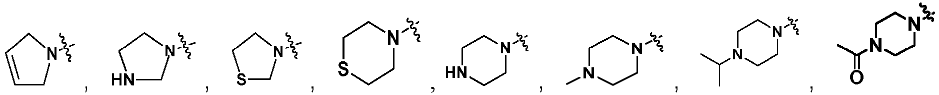 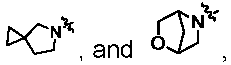
wherein the C₂₋₉ heterocyclyl, the C₆₋₁₄ heterospirocyclyl, the C₆₋₁₄ cross-linked heterocyclyl, or the C₄₋₁₀ heteroaryl may be unsubstituted or substituted with one or more substituents selected from hydrogen, halogen, nitro, cyano, haloC₁₋₆ alkyl, C₁₋₆ alkyl, C₂₋₆ alkenyl, C₂₋₆ alkynyl, C₁₋₆ alkoxy, -C(O)R₁₁, haloC₁₋₆ alkoxy, and C₂₋₆ heterocyclyl.

In an embodiment,
R₁ may be methyl, ethyl, propyl, or isopropyl;
R₂ and R₃ may be hydrogen;
R₄ and R₅ may be linked to each other to form, with the amide nitrogen of Formula 1, a C₂₋₉ heterocyclyl, C₆₋₁₄ heterospirocyclyl, C₆₋₁₄ cross-linked heterocyclyl, or C₄₋₁₀ heteroaryl, which is one of 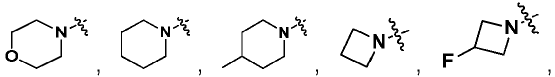 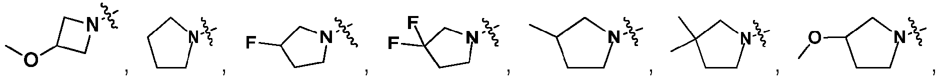 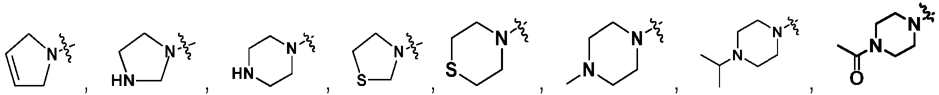 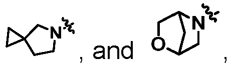
wherein the C₂₋₉ heterocyclyl, the C₆₋₁₄ heterospirocyclyl, the C₆₋₁₄ cross-linked heterocyclyl, or the C₄₋₁₀ heteroaryl may be unsubstituted or substituted with at least one substituent selected from hydrogen, halogen, C₁₋₆ alkyl, and -C(O)(C₁₋₆ alkyl).

In some embodiments, C₂₋₉ heterocyclyl, C₆₋₁₄ heterospirocyclyl, C₆₋₁₄ cross-linked heterocyclyl, or C₄₋₁₀ heteroaryl may be unsubstituted or substituted with at least one substituent selected from hydrogen, halogen, methyl, ethyl, propyl, butyl, isopropyl, isobutyl, and acetyl.

In an embodiment,
the compound may be a compound selected from compounds of Formula 2, optical isomers, diastereomers, solvates, and hydrates thereof, and pharmaceutically acceptable salts thereof. 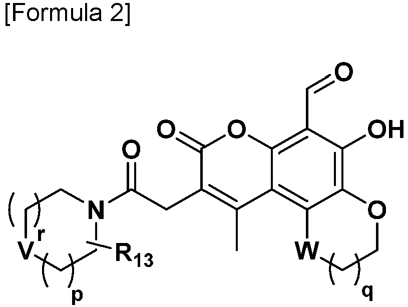 wherein, in Formula 2,
V and W may each independently be -CH₂-, -S-, -O-, or -NR₁₄-;
p, q, and r may each independently be an integer from 0 or 1;
R₁₃ may be hydrogen, halogen, nitro, cyano, haloC₁₋₆ alkyl, C₁₋₆ alkyl, C₂₋₆ alkenyl, C₂₋₆ alkynyl, C₁₋₆ alkoxy, or haloC₁₋₆ alkoxy; and
each R₁₄ may independently be hydrogen, C₁₋₆ alkyl, haloC₁₋₆ alkyl, or -C(O)(C₁₋₆ alkyl).

In an embodiment,
R₁₃ may be hydrogen, halogen, methyl, ethyl, propyl, butyl, or isopropyl.

In an embodiment,
R₁₄ may be hydrogen, halogen, methyl, ethyl, propyl, butyl, isopropyl, or acetyl.

In an embodiment, the compound may be selected from the group consisting of the following compounds, optical isomers, diastereomers, solvates, and hydrates thereof, and pharmaceutically acceptable salts of these:
1) 4-hydroxy-9-methyl-8-(2-morpholino-2-oxoethyl)-7-oxo-1,7-dihydro-2H-furo[3,2-*f*]chromene-5-carbaldehyde;
2) 4-hydroxy-9-methyl-8-(2-morpholino-2-oxoethyl)-7-oxo-7*H*-[1,3]dioxolo[4,5-flchromene-5-carbaldehyde;
3) 6-hydroxy-1-methyl-2-(2-morpholino-2-oxoethyl)-3-oxo-3,8,9,10-tetrahydropyrano[3,2-*f*]chromene-5-carbaldehyde;
4) 6-hydroxy-1-methyl-2-(2-(4-methylpiperidin-1-yl)-2-oxoethyl)-3-oxo-3,8,9,10-tetrahydropyrano[3,2-*f*]chromene-5-carbaldehyde;
5) 6-hydroxy-1-methyl-3-oxo-2-(2-oxo-2-(pyrrolidin-1-yl)ethyl)-3,8,9,10-tetrahydropyrano[3,2-*f*]chromene-5-carbaldehyde;
6) 6-hydroxy-1-methyl-2-(2-(4-methylpiperazin-1-yl)-2-oxoethyl)-3-oxo-3,8,9,10-tetrahydropyrano[3,2-*f*]chromene-5-carbaldehyde;
7) 6-hydroxy-2-(2-(4-isopropylpiperazin-1-yl)-2-oxoethyl)-1-methyl-3-oxo-3,8,9,10-tetrahydropyrano[3,2-*f*]chromene-5-carbaldehyde;
8) *N*-ethyl-2-(5-formyl-4-hydroxy-9-methyl-7-oxo-1,7-dihydro-2H-furo[3,2-*f]*chromen-8-yl)acetamide;
9) *N,N*-diethyl-2-(5-formyl-4-hydroxy-9-methyl-7-oxo-1,7-dihydro-2H-furo[3,2-*f*]chromen-8-yl)acetamide;
10) 2-(2-(3,3-difluoropyrrolidin-1-yl)-2-oxoethyl)-6-hydroxy-1-methyl-3-oxo-3,8,9,10-tetrahydropyrano[3,2-*f*]chromene-5-carbaldehyde;
11) 6-hydroxy-1-methyl-2-(2-(3-methylpyrrolidin-1-yl)-2-oxoethyl)-3-oxo-3,8,9,10-tetrahydropyrano[3,2-*f*]chromene-5-carbaldehyde;
12) 2-(2-(3,3-dimethylpyrrolidin-1-yl)-2-oxoethyl)-6-hydroxy-1-methyl-3-oxo-3,8,9,10-tetrahydropyrano[3,2-*f*]chromene-5-carbaldehyde;
13) 2-(2-(azetidin-1-yl)-2-oxoethyl)-6-hydroxy-1-methyl-3-oxo-3,8,9,10-tetrahydropyrano[3,2-*f*]chromene-5-carbaldehyde;
14) 6-hydroxy-2-(2-(3-methoxypyrrolidin-1-yl)-2-oxoethyl)-1-methyl-3-oxo-3,8,9,10-tetrahydropyrano[3,2-*f*]chromene-5-carbaldehyde;
15) 6-hydroxy-2-(2-(3-methoxyazetidin-1-yl)-2-oxoethyl)-1-methyl-3-oxo-3,8,9,10-tetrahydropyrano[3,2-*f*]chromene-5-carbaldehyde;
16) 2-(2-(3-fluoropyrrolidin-1-yl)-2-oxoethyl)-6-hydroxy-1-methyl-3-oxo-3,8,9,10-tetrahydropyrano[3,2-*f*]chromene-5-carbaldehyde;
17) *N-*ethyl-2-(5-formyl-6-hydroxy-1-methyl-3-oxo-3,8,9,10-tetrahydropyrano[3,2-*f*]chromen-2-yl)acetamide;
18) 1-ethyl-6-hydroxy-3-oxo-2-(2-oxo-2-(pyrrolidin-1-yl)ethyl)-3,8,9,10-tetrahydropyrano[3,2-*f*]chromene-5-carbaldehyde;
19) 2-(2-(3-fluoroazetidin-1-yl)-2-oxoethyl)-6-hydroxy-1-methyl-3-oxo-3,8,9,10-tetrahydropyrano[3,2-*f*]chromene-5-carbaldehyde;
20) 6-hydroxy-1-methyl-3-oxo-2-(2-oxo-2-(5-azaspiro[2.4]heptan-5-yl)ethyl)-3,8,9,10-tetrahydropyrano[3,2-*f*]chromene-5-carbaldehyde;
21) 6-hydroxy-1-methyl-3-oxo-2-(2-oxo-2-(piperidin-1-yl)ethyl)-3,8,9,10-tetrahydropyrano[3,2-*f*]chromene-5-carbaldehyde;
22) 2-(5-formyl-6-hydroxy-1-methyl-3-oxo-3,8,9,10-tetrahydropyrano[3,2-*f*]chromen-2-yl)acetamide;
23) 6-hydroxy-1-isopropyl-3-oxo-2-(2-oxo-2-(pyrrolidin-1-yl)ethyl)-3,8,9,10-tetrahydropyrano[3,2-*f*]chromene-5-carbaldehyde;
24) 2-(5-formyl-6-hydroxy-1-methyl-3-oxo-3,8,9,10-tetrahydropyrano[3,2-*f*]chromen-2-yl)-*N*-(2-methoxyethyl)acetamide;
25) *N,N*-diethyl-2-(5-formyl-6-hydroxy-1-methyl-3-oxo-3,8,9,10-tetrahydropyrano[3,2-*f*]chromen-2-yl)acetamide;
26) 2-(2-(4-acetylpiperazin-1-yl)-2-oxoethyl)-6-hydroxy-1-methyl-3-oxo-3,8,9,10-tetrahydropyrano[3,2-*f*]chromene-5-carbaldehyde;
27) 2-(2-(2-oxa-5-azabicyclo[2.2.1]heptan-5-yl)-2-oxoethyl)-6-hydroxy-1-methyl-3-oxo-3,8,9,10-tetrahydropyrano[3,2-*f*]chromene-5-carbaldehyde;
28) 6-hydroxy-1-methyl-3-oxo-2-(2-oxo-2-thiomorpholinoethyl)-3,8,9,10-tetrahydropyrano[3,2-*f*]chromene-5-carbaldehyde;
29) 2-(5-formyl-6-hydroxy-1-methyl-3-oxo-3,8,9,10-tetrahydropyrano[3,2-*f*]chromen-2-yl)-*N*-(2-morpholinoethyl)acetamide;
30) 6-hydroxy-1-methyl-3-oxo-2-(2-oxo-2-(thiazolidin-3-yl)ethyl)-3,8,9,10-tetrahydropyrano[3,2-*f*]chromene-5-carbaldehyde; and
31) 6-hydroxy-1-methyl-3-oxo-2-(2-oxo-2-(piperazin-1-yl)ethyl)-3,8,9,10-tetrahydropyrano[3,2-*f*]chromene-5-carbaldehyde.

Another embodiment of the present disclosure provides a pharmaceutical composition for treating or preventing inositol requiring enzyme 1α (IRE1α)-associated diseases including, as an active ingredient, a compound selected from compounds of Formula 1, optical isomers, diastereomers, solvates, and hydrates thereof, and pharmaceutically acceptable salts thereof.

In an embodiment, the composition may exhibit IRE1α inhibitory activity.

In an embodiment, the composition exhibits IRE1α inhibitory activity and is for treating cancers or tumors that are treatable by IRE1α inhibitory activity.

The compound or composition according to an embodiment may exhibit IRE1α inhibitory activity, and may be used to inhibit IRE1α activity, for example, RNA or mRNA cleavage or RNA or mRNA splicing.

In an embodiment, the cancer may be selected from the group consisting of breast cancer, liver cancer, ovarian carcinoma, pancreatic cancer, head and neck cancer, non-small cell lung cancer, glioblastoma, and multiple myeloma, but is not limited thereto. In an embodiment, the pharmaceutical composition may include a therapeutically effective amount of a compound selected from compounds of Formula 1, optical isomers, diastereomers, solvates, and hydrates thereof, and pharmaceutically acceptable salts thereof.

The term "therapeutically effective amount" as used herein refers to an amount of the compound of the disclosure that treats or prevents a particular disease, condition, or disorder, attenuates, ameliorates, or eliminates one or more symptoms of a particular disease, condition, or disorder, or prevents or delays the onset of one or more symptoms of a particular disease, condition, or disorder.

A physician skilled in the art may easily determine and prescribe an effective, required dose for a pharmaceutical composition. For example, the pharmaceutical composition may include 0.0001 mg to 10 g of the compound, but is not limited thereto.

In an embodiment, the pharmaceutical composition may further include a pharmaceutically acceptable additive in addition to the active ingredient. The additive may be, for example, a diluent, a disintegrant, a binder, a lubricant, a surfactant, a suspending agent, or an emulsifier, but is not limited thereto.

The pharmaceutical composition of the present disclosure may be formulated according to conventional methods, and may be administered in various oral dosage forms, such as tablets, pills, powders, capsules, syrups, emulsions, and microemulsions, or in a parenteral dosage form, such as intramuscular, intravenous or subcutaneous administration.

Another embodiment of the present disclosure provides a treatment method of administering, to a subject suffering from an IRE1α-associated disease, a pharmaceutical composition including, as an active ingredient, a compound selected from compounds of Formula 1, optical isomers, diastereomers, solvates, and hydrates thereof, and pharmaceutically acceptable salts thereof.

The term "treating" or "treatment" as used herein refers to inhibiting a disease, e.g., inhibiting a disease, condition, or disorder, i.e., preventing or reversing further development of the pathology and/or symptoms, or ameliorating the disease, e.g., reducing the severity of the disease, in a subject experiencing or exhibiting pathology or symptoms of the disease, condition, or disorder.

The term "preventing" or "prevention" refers to preventing a disease, e.g., the prevention of a disease, condition, or disorder in a subject who may have a predisposition to a disease, pathology, or disorder, but has not yet experienced or exhibited the pathology or symptoms of the disease.

The term "subject" or "individual" as used herein may be a vertebrate such as a mammal, fish, bird, reptile or amphibian. For example, the subject may be a human, non-human primate, horse, pig, rabbit, dog, sheep, goat, cow, cat, guinea pig or rodent.

As used herein, the terms "administering" and "administration" refer to any method of providing a disclosed composition to a subject.

The dosage, frequency of administration, or method of administration of the compound or pharmaceutical composition according to an embodiment may vary depending on the subject to be treated, the severity of the disease or condition, the speed of administration, and the judgment of the prescribing physician. For example, a dose for a person weighing 70 kg may be administered in an amount of 0.0001 mg to 10 g per day, for example, 1 mg to 1 g per day. The number of administrations may be one to several times, such as 1 to 4 times, or an on/off schedule, and the administration method may use an oral or parenteral route. For example, the compound or pharmaceutical composition according to an embodiment may be administered through an oral or parenteral route in an amount ranging from 0.1 mg/kg to 100 mg/kg (body weight).

A physician may gradually increase the dose of a compound or pharmaceutical composition of the present disclosure administered to a subject, starting from a level lower than that necessary to achieve the target therapeutic effect to a level at which the intended effect is achieved.

Another embodiment of the present disclosure provides a kit including, as an active ingredient, a compound selected from compounds of Formula 1, optical isomers, diastereomers, solvates, and hydrates thereof, and pharmaceutically acceptable salt thereof.

In an embodiment, the therapeutic agent may be a drug for treating an IRE1α-associated disease, such as a drug for treating cancer. For example, the therapeutic agent may be a chemotherapeutic drug for the treatment of cancer.

In an embodiment, the compound, composition, and kit of the present disclosure may be administered alone or simultaneously with at least one other therapeutic agent, separately or sequentially.

In the context of this disclosure, singular forms of a word may include the plural and vice versa unless the context clearly dictates otherwise.

Numerical values described herein are considered to include the meaning of "about" even if not explicitly stated. The term "about" as used herein refers to a given value or range within 5%, for example, the range from 1% to 2%.

The numerical range expressed using the term "to" refers to a range including the numerical values described before and after the term "to" as the lower limit and the upper limit, respectively.

The terms "has", "may have", "includes", or "may include" indicate the presence of a corresponding feature (e.g., a constituent, such as a numerical value or a component), does not exclude the presence of additional features.

The contents of all publications cited herein by reference are hereby incorporated by reference in their entirety.

Hereinafter, a method of preparing the compound of Formula 1 will be described in detail.

The compound of Formula 1 according to an embodiment of the present disclosure may be more easily prepared by the method of Reaction Scheme 3 after synthesizing an intermediate according to the synthetic methods shown in Reaction Scheme 1 and Reaction Scheme 2.

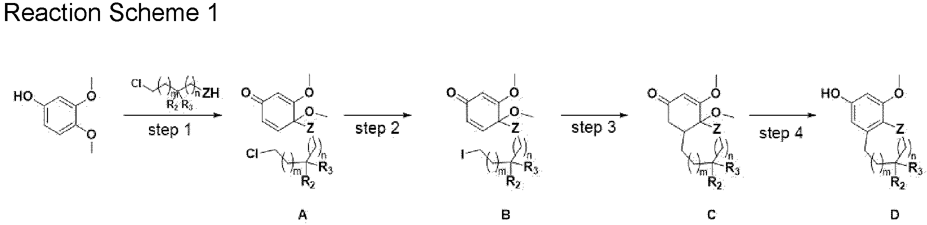

### [Step-1]

(Diacetoxyiodo)benzene (1.1 eq.) and potassium carbonate (1.2 eq.) are added to haloalcohol or haloamine. 3,4-dimethoxyphenol (1 eq., standard eq.) is slowly added dropwise thereto. After completion of the dropwise addition, the mixture is stirred at room temperature, and when the reaction is completed, the reaction solvent is distilled off under reduced pressure, sodium bicarbonate saturated solution is added thereto, and extraction is performed using ethyl acetate. The organic layer is dried using anhydrous sodium sulfate and filtered under reduced pressure, and the filtered organic layer is concentrated under reduced pressure. The obtained residue is purified by MPLC (ethyl acetate:hexane) to obtain Compound A, which is the target compound.

### [Step-21

Compound A (1 eq., standard eq.) prepared in [Step-1] and sodium iodide (10 eq.) are added to acetone, and then the mixture is stirred under reflux overnight. After completion of the reaction, acetone is removed by distillation under reduced pressure, water is added thereto, and extraction is performed using dichloromethane. The organic layer is dried using anhydrous sodium sulfate and filtered under reduced pressure, and the filtered organic layer is concentrated under reduced pressure. The obtained residue is purified by MPLC (ethyl acetate:hexane) to obtain Compound B.

### [Step-3]

Compound B (1 eq., standard eq.) obtained in [Step-2] is dissolved in toluene, and azobisisobutyronitrile (1 eq.) is added thereto. Tri-n-butyltin hydride (Bu₃SnH) (5 eq.) is dissolved in toluene, and then added dropwise thereto, and the reaction solution is stirred at 90°C overnight. After the reaction is completed, toluene is removed by distillation under reduced pressure, and water is added thereto to extract with ethyl acetate. The organic layer is dried using anhydrous sodium sulfate and filtered under reduced pressure, and the filtered organic layer is concentrated under reduced pressure. The obtained residue is purified by MPLC (ethyl acetate:hexane) to obtain Compound C.

### [Step-4]

Compound C (1 eq., standard eq.) obtained in [Step-3], p-toluenesulfonic acid (0.5 eq.), and 4A Molecular Sieve (5v/v) are added to dichloromethane and stirred at room temperature. After the reaction is complete, the reaction solution is filtered through a filter filled with celite and washed with dichloromethane. The filtered organic layer is concentrated under reduced pressure and the obtained residue is purified by MPLC (ethyl acetate:hexane) to obtain Compound D.

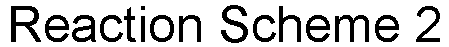

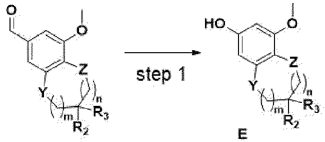

### [Step-1]

3-methoxy-1,3-benzodioxole-5-carbaldehyde (1 eq., standard eq.) is dissolved in dichloromethane, and the temperature thereof is lowered to 0 °C. 3-chloroperoxybenzoic acid (2 eq.) is added to the reaction solution to which sodium bicarbonate (2 eq.) has been added, followed by stirring overnight at room temperature. After confirming that the reaction is complete, methanol and a saturated deuterium solution are added thereto and stirred at room temperature, and the resulting solid is dissolved in water, and then methanol is distilled off under reduced pressure. The reaction solution is extracted using dichloromethane, and the organic layer is dried using anhydrous sodium sulfate and then concentrated under reduced pressure. The obtained residue is purified by MPLC (ethyl acetate:hexane) to obtain Compound E.

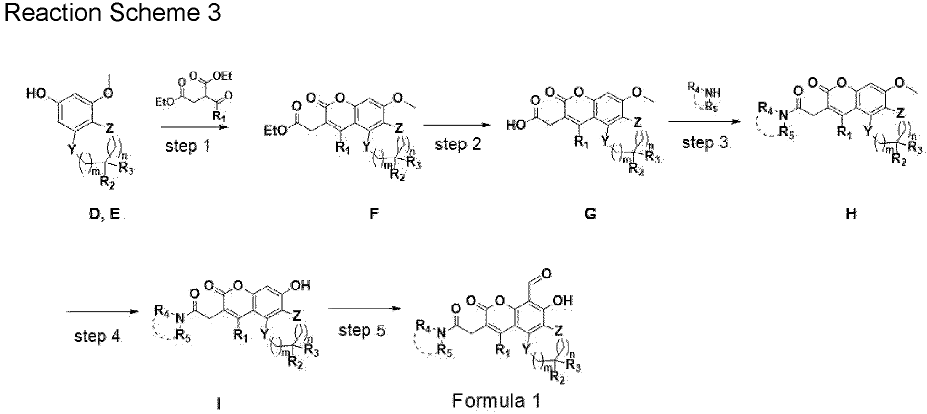

### [Step-1]

Compound D or Compound E (1 eq., standard eq.) obtained in Reaction Scheme 1 and Reaction Scheme 2 and diethylacetyl succinate (1.2 eq.) are added to methanesulfonic acid. After the reaction solution is stirred at room temperature overnight to complete the reaction, water is added thereto, followed by extraction using ethyl acetate. The organic layer is dried using anhydrous sodium sulfate and filtered under reduced pressure, and the filtered organic layer is concentrated under reduced pressure. The obtained residue is purified by MPLC (ethyl acetate:hexane) to obtain Compound F.

### [Step-2]

Compound F (1 eq., standard eq.) obtained in [Step-1] is dissolved in tetrahydrofuran/methanol/water and sodium hydroxide (5 eq.) is added thereto. The reaction solution is stirred at room temperature. When the reaction is complete, acidifying is performed using 6N hydrochloric acid until the pH of the resulting solution reached to be in the range of pH 3 to pH 4 to obtain a solid product. The obtained solid is filtered under reduced pressure and the filtered solid is washed with water. The filtered solid is dried to obtain Compound G.

### [Step-3]

Compound G obtained in [Step-2] (1 eq., standard eq.), R₄R₅ amine (1.2 eq.) and (benzotriazol-1-yloxy) tripyrrolidinophosphonium hexafluorophosphate (1.2 eq.) are dissolved in DMF, and N,N-diisopropylethylamine (1.2 eq.) is slowly added thereto. After the reaction solution is stirred at room temperature overnight to complete the reaction, water is added thereto, followed by extraction using ethyl acetate. The organic layer is dried using anhydrous sodium sulfate and filtered under reduced pressure, and the filtered organic layer is concentrated under reduced pressure. The obtained residue is purified by MPLC (ethyl acetate:hexane) to obtain Compound H.

### [Step-4]

Compound H (1 eq., standard eq.) obtained in [Step-3] is dissolved in dichloromethane, and a solution of boron tribromide (BBr₃, 7 eq.) is slowly added dropwise thereto at room temperature. After the reaction is completed by stirring the reaction solution at room temperature, water is added thereto and the extraction process is performed using a methanol/dichloromethane solution. The organic layer is dried using anhydrous sodium sulfate and filtered under reduced pressure, and the filtered organic layer is concentrated under reduced pressure. The obtained residue is purified by MPLC (dichloromethane:methanol) to obtain Compound I.

### [Step-51

Compound I (1 eq., standard eq.) obtained in [Step-4] and hexamethylenetetramine (HMTA, 4 eq.) are dissolved in trifluoroacetic acid, and stirred at 90 °C. After the reaction is completed, the reaction product is cooled to room temperature and then, neutralized by adding aqueous sodium bicarbonate solution dropwise thereto. The resultant product is extracted using a methanol/dichloromethane solution, dried using anhydrous sodium sulfate, and then concentrated under reduced pressure. The obtained residue is purified by MPLC (dichloromethane:methanol) to obtain Formula 1.

In Reaction Scheme 1 to Reaction Scheme 3, R₁, R₂, R₃, R₄, R₅, Y, Z, n and m are as defined in connection with Formula 1, but are not limited thereto.

The compound of Formula 1 according to an embodiment of the present disclosure may be prepared according to the methods shown in Reaction Scheme 1 to Reaction Scheme 3, but is not limited thereto. Those skilled in the art of organic compounds may appropriately adjust specific reaction pathways, reaction conditions, reaction amounts, and the like.

Hereinafter, the present disclosure will be described in more detail by the following Examples and Experimental Examples. However, these Examples and Experimental Examples are only for helping understanding of the present disclosure, and the scope of the present disclosure is not limited by these in any sense.

### Example 1: 4-hydroxy-9-methyl-8-(2-morpholino-2-oxoethyl)-7-oxo-1,7-dihydro-2H-furo[3,2-f]chromene-5-carbaldehyde

### [Step-1] Preparation of 4-(2-chloroethoxy)-3,4-dimethoxycyclohexa-2,5-dien-1-one

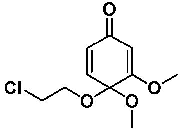

(diacetoxyiodo)benzene (Phl(OAc)₂ (25.3 g, 78.55 mmol) and potassium carbonate (11.8 g, 85.38 mmol) were added to 200 mL of 2-chloroethanol, and then, 3,4-dimethoxyphenol (11 g, 71.35 mmol) was dissolved in 100 mL of 2-chloroethanol and added dropwise thereto slowly at room temperature. After the dropwise addition was completed, the mixture was stirred at room temperature for 3 hours. After the reaction was completed, 2-chloroethanol was removed by distillation under reduced pressure. 300 mL of sodium bicarbonate saturated solution was added to the reaction solution and extracted three times using ethyl acetate, and the organic layer was dried using anhydrous sodium sulfate. The organic layer filtered under reduced pressure was concentrated under reduced pressure, and the obtained residue was purified by MPLC (ethyl acetate:hexane = 1:4 (v/v) to 1:1 (v/v)) to obtain 15 g (90%, yield) of the target compound.

¹H-NMR (300 MHz, CDCl₃): *δ*6.59 (d, *J= 10.2* Hz, 1H), 6.29 (dd, J= 10.2, 3.7 Hz, 1H), 5.62 (d, *J=* 1.8 Hz, 1H), 3.81 (s, 3H), 3.78-3.74 (m, 2H), 3.63-3.58 (m, 2H), 3.34 (s, 3H).

### [Step-2] Preparation of 4-(2-iodoethoxy)-3,4-dimethoxycyclohexa-2,5-dien-1-one

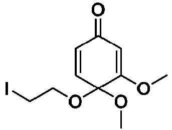

4-(2-chloroethoxy)-3,4-dimethoxycyclohexa-2,5-dien-1-one prepared in [Step-1] (15 g, 64.47 mmol) and sodium iodide (96.64 g, 644.72 mmol) were added to 300 mL (4.35 g, 108.63 mmol) of acetone and stirred under reflux for 48 hours. After the reaction was completed, acetone was removed by distillation under reduced pressure, and 200 mL of water was added thereto, followed by extraction using dichloromethane three times. The organic layer was dried using anhydrous sodium sulfate and filtered under reduced pressure, and the filtered organic layer was concentrated under reduced pressure. The residue obtained was purified by MPLC (ethyl acetate:hexane = 1:1 (v/v)) to obtain 17 g (81%, yield) of the target compound.

¹H-NMR (300 MHz, CDCl₃): *δ*6.62 (d, *J=* 10.2 Hz, 1H), 6.32 (dd, *J*= 10.2, 1.8 Hz, 1H), 5.64 (d, *J=* 1.8 Hz, 1H), 3.81 (s, 3H), 3.79-3.75 (m, 2H), 3.37 (s, 3H), 3.31-3.23 (m, 2H).

### [Step-3] Preparation of 7,7a-dimethoxy-2,3,3a,7a-tetrahydrobenzofuran-5(4H)-one

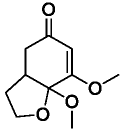

4-(2-iodoethoxy)-3,4-dimethoxycyclohexa-2,5-dien-1-one (17 g, 52.45 mmol) obtained in [Step-2] was dissolved in 300 mL of toluene, and azobisisobutyronitrile (AIBN, 8.6 g, 52.45 mmol) was added thereto. Tri-n-butyl tin hydride (Bu₃SnH, 76.33 g, 262.26 mmol) was dissolved in 50 mL toluene and added dropwise for 30 min, then the reaction was stirred at 90 °C overnight. After the reaction was completed, toluene was removed by distillation under reduced pressure, and water was removed therefrom, and in this state, extraction was performed three times using ethyl acetate. The organic layer was dried using anhydrous sodium sulfate and filtered under reduced pressure, and the filtered organic layer was concentrated under reduced pressure. The residue obtained was purified by MPLC (ethyl acetate:hexane = 1:3 (v/v)) to obtain 4.7 g (45%, yield) of the target compound.

¹H-NMR (300 MHz, CDCl₃): *δ*5.38 (s, 1H), 4.16-4.09 (m, 2H), 3.77 (s, 3H), 3.38 (s, 3H) 2.85-2.78 (m, 1H), 2.59-2.57(m, 1H), 2.47-2.39(m, 1H), 2.18-2.06 (m, 1 H), 1.69-1.81 (m, 1H)

### [Step-4] Preparation of 7-methoxy-2,3-dihydrobenzofuran-5-ol

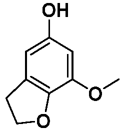

7,7a-dimethoxy-2,3,3a,7a-tetrahydrobenzofuran-5(4H)-one (4.2 g, 21.19 mmol) obtained in [Step-3] and p-toluenesulfonic acid (2.02 g, 10.59 mmol) and 4A Molecular Sieve (20 g) were added to 200 mL of dichloromethane, and the mixture was stirred at room temperature for 3 hours. After the reaction was completed, the reaction solution was filtered through a filter filled with celite and washed with 200 mL of dichloromethane. The filtered organic layer was concentrated under reduced pressure, and the obtained residue was purified by MPLC (ethyl acetate:hexane = 1:4 (v/v) to 1:1 (v/v)) to obtain 3.2 g (91%, yield) of the target compound.

¹H-NMR (300 MHz, CDCl₃): *δ*6.35-6.32 (m, 2H), 4.69 (brs, 1H), 4.58 (t, *J=* 8.7 Hz, 2H), 3.82 (s, 3H), 3.17 (t, *J=* 8.7 Hz, 2H).

### [Step-5] Preparation of ethyl 2-(4-methoxy-9-methyl-7-oxo-1,7-dihydro-2H-furo[3,2-f]chromen-8-yl)acetate

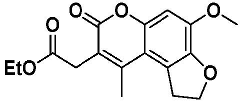

7-methoxy-2,3-dihydrobenzofuran-5-ol (520 mg, 3.13 mmol) prepared in [Step-4] and diethylacetylsuccinate (855 mg, 3.76 mmol) were added to 5 mL of methanesulfonic acid. The reaction solution was stirred overnight at room temperature. After the reaction was completed, 100 mL of water was added and extracted three times with ethyl acetate. The organic layer was dried using anhydrous sodium sulfate and filtered under reduced pressure, and the filtered organic layer was concentrated under reduced pressure. The obtained residue was purified by MPLC (ethyl acetate:hexane = 1:3 (v/v)) to obtain 780 mg of the target compound (78%, yield).

¹H-NMR (300 MHz, DMSO-*d₆*): *δ* 6.97 (s, 1H), 4.62-4.56 (m, 2H), 4.12-4.02 (m, 2H), 3.86 (s, 3H), 3.71-3.63 (m, 4H), 2.42 (s, 3H), 1.21-1.16 (m, 3H).

### [Step-6] Preparation of 2-(4-methoxy-9-methyl-7-oxo-1,7-dihydro-2H-furo[3,2-f]chromen-8-yl)acetic acid

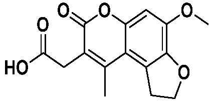

ethyl 2-(4-methoxy-9-methyl-7-oxo-1,7-dihydro-2H-furo[3,2-*f*]chromen-8-yl)acetate (780 mg, 2.45 mmol) obtained in [Step-5] was dissolved in a mixture including tetrahydrofuran/methanol/water = 20 mL/5 mL/5 mL and sodium hydroxide (490 mg, 12.25 mmol) was added thereto. After stirring the reaction solution at room temperature for 3 hours, when the reaction was complete, acidification was performed using 6N hydrochloric acid until the pH of the resulting solution reached to be in the range of pH 3 to pH 4 to obtain a solid product. The obtained solid was filtered under reduced pressure and the filtered solid was washed with distilled water. The filtered solid was dried in an oven drier at 55 °C to obtain 510 mg (72%, yield) of the target compound.

¹H-NMR (300 MHz, DMSO-*d₆*): *δ* 6.96 (s, 1H), 4.61-4.56 (m, 2H), 3.86 (s, 3H), 3.71-3.65 (m, 2H), 3.59 (s, 2H), 2.42 (s, 3H).

### [Step-7] Preparation of 4-methoxy-9-methyl-8-(2-morpholino-2-oxoethyl)-1,2-dihydro-7H-furo[3,2-f]chromen-7-one

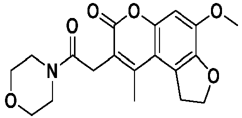

2-(4-methoxy-9-methyl-7-oxo-1,7-dihydro-2H-furo[3,2-t]chromen-8-yl)acetic acid obtained in [Step-6] (510 mg, 1.76 mmol), morpholine (183 mg, 2.11 mmol) and (benzotriazol-1-yloxy)tripyrrolidinophosphonium hexafluorophosphate (PyBOP, 1.1 g, 2.11 mmol) were dissolved in 30 mL of DMF, and *N,N-*diisopropylethylamine (273 mg, 2.11 mmol) was added slowly thereto. After the reaction solution was stirred at room temperature overnight to complete the reaction, 100 mL of water was added thereto, followed by extraction using ethyl acetate three times. The organic layer was dried using anhydrous sodium sulfate and filtered under reduced pressure, and the filtered organic layer was concentrated under reduced pressure. The obtained residue was purified by MPLC (ethyl acetate:hexane = 1:1 (v/v)) to obtain 470 mg (74%, yield) of the target compound.

¹H-NMR (300 MHz, DMSO-*d₆*): *δ* 6.95 (s, 1H), 4.61-4.55 (m, 2H), 3.69 (s, 3H), 3.67-3.56 (m, 10H), 3.45-3.42 (m, 2H), 2.36 (s, 3H).

### [Step-8] Preparation of 4-hydroxy-9-methyl-8-(2-morpholino-2-oxoethyl)-1,2-dihydro-7H-furo[3,2-f]chromen-7-one

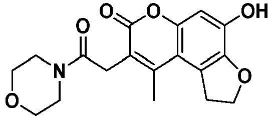

4-methoxy-9-methyl-8-(2-morpholino-2-oxoethyl)-1,2-dihydro-7*H*-furo[3,2-flchromen-7-one (300 mg, 0.83 mmol) obtained in [Step-7] was dissolved in 10 mL of dichloromethane, and a boron tribromide solution 1.0 M in tetrahydrofuran (BBr₃, 5.84 mL) was slowly added dropwise thereto at room temperature. After the reaction was completed by stirring at room temperature for 4 hours, the mixture was cooled at 0 °C, and 10 mL of water was added thereto, followed by extraction using a 20% methanol/dichloromethane solution three times. The organic layer was dried using anhydrous sodium sulfate and filtered under reduced pressure, and the filtered organic layer was concentrated under reduced pressure. The obtained residue was purified by MPLC (dichloromethane: methanol = 10: 1 (v/v)) to obtain 200 mg (69%, yield) of the target compound.

¹H-NMR (300 MHz, CDCl₃): *δ* 6.48 (s, 1H), 4.29-4.25 (m, 2H), 3.76-3.65 (m, 10H), 3.18-3.14 (m, 2H), 2.57 (s, 3H)

### [Step-9] Preparation of 4-hydroxy-9-methyl-8-(2-morpholino-2-oxoethyl)-7-oxo-1,7-dihydro-2H-furo[3,2-f]chromene-5-carbaldehyde

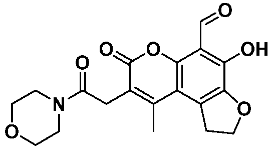

4-hydroxy-9-methyl-8-(2-morpholino-2-oxoethyl)-1,2-dihydro-7*H*-furo[3,2-flchromen-7-one (200 mg, 0.57 mmol) obtained in [Step-8] and hexamethylenetetramine (HMTA, 328 mg, 2.31 mmol) were dissolved in 10 mL of trifluoroacetic acid, and the mixture was stirred at 90°C for 2 hours. After the reaction was completed, the reaction product was cooled to room temperature and then, neutralized by adding aqueous sodium bicarbonate solution dropwise thereto. The resultant product was extracted three times using a 20% methanol/dichloromethane solution, dried using anhydrous sodium sulfate, and then filtered under reduced pressure. The filtered organic layer was concentrated under reduced pressure, and the resulting residue was purified by MPLC (dichloromethane:methanol=20:1 (v/v)) to obtain 10 mg (5%, yield) of the target compound.

¹H-NMR (300 MHz, CD₃OD): *δ* 10.42 (s, 1H), 4.67-4.61 (m, 2H), 3.76-3.66 (m, 10H), 3.61-3.58 (m, 2H), 2.41 (s, 3H).

MS (ESI⁺, m/z): 374.2 [M+H]⁺

### Example 2: 4-hydroxy-9-methyl-8-(2-morpholino-2-oxoethyl)-7-oxo-7H-[1,3]dioxolo[4,5-f]chromene-5-carbaldehyde

### [Step 1] Preparation of 7-methoxybenzo[d][1,3]dioxol-5-ol

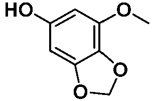

7-methoxy-1,3-benzodioxole-5-carbaldehyde (5 g, 27.75 mmol, eNovation) was dissolved in 143 mL of dichloromethane, and the temperature thereof was lowered to 0 °C, and sodium bicarbonate (4.66 g, 55.48 mmol) was added thereto. 3-chloroperoxybenzoic acid (mCPBA, 9.58 g, 55.51 mmol) was added to the reaction mixture, and then, the mixture was stirred at room temperature overnight. After confirming that the reaction was complete, 143 mL of methanol was added and 72 mL of saturated deuterium solution was added, followed by additional stirring for 1 hour. The resulting solid was dissolved in water, methanol was concentrated under reduced pressure, the reaction solution was extracted 3 times with dichloromethane, and the organic layer was dried using anhydrous sodium sulfate and filtered under reduced pressure. The filtered organic layer was concentrated under reduced pressure, and the resulting residue was purified by MPLC (ethyl acetate:hexane=1:4 (v/v)) to obtain 3.5 g (75%, yield) of the target compound.

¹H-NMR (300 MHz, CDCl₃): *δ* 6.13-6.07 (m, 2H), 3.86 (s, 2H), 2.32 (s, 3H).

### [Step 2] Preparation of ethyl 2-(4-methoxy-9-methyl-7-oxo-7H-[1,3]dioxolo[4,5-f]chromen-8-yl)acetate

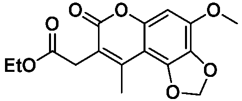

310 mg (16%, yield) of the target compound was obtained in the same manner as in step 5) of Example 1, except that 7-methoxybenzo[d][1,3]dioxole-5-ol (1 g, 5.95 mmol) prepared in step 1 of the present example, was used instead of 7-methoxy-2,3-dihydrobenzofuran-5-ol in step 5 of Example 1.

¹H-NMR (300 MHz, CDCl₃): *δ* 6.53 (s, 1H), 6.09 (s, 2H), 4.23-4.16 (m, 2H), 3.95 (s, 3H), 3.69 (s, 2H), 2.49 (s, 3H), 1.31-1.27 (m, 3H).

### [Step 3] Preparation of 2-(4-methoxy-9-methyl-7-oxo-7H-[1,3]dioxolo[4,5-flchromen-8-yl)acetic acid

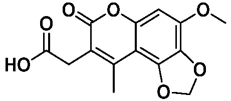

160 mg (57%, yield) of the target compound was obtained in the same manner as in step 6) of Example 1, except that ethyl 2-(4-methoxy-9-methyl-7-oxo-7H-[1,3]dioxolo[4,5-*f*]chromen-8-yl)acetate (310 mg, 5.95 mmol) prepared in step 2 of the present example was used instead of ethyl 2-(4-methoxy-9-methyl-7-oxo-1,7-dihydro-2*H*-furo[3,2-*f*]chromen-8-yl)acetate in step 6) of Example 1.

¹H-NMR (300 MHz, CD₃OD): *δ* 6.66 (s, 1H), 6.11 (s, 2H), 3.96 (s, 3H), 3.67 (s, 2H), 2.52 (s, 3H).

### [Step 4] Preparation of 4-methoxy-9-methyl-8-(2-morpholino-2-oxoethyl)-7H-[1,3]dioxolo[4,5-f]chromen-7-one

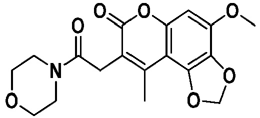

425 mg (71%, yield) of the target compound was obtained in the same manner as in step 7) of Example 1, except that 2-(4-methoxy-9-methyl-7-oxo-7*H-*[1,3]dioxolo[4,5-*f*]chromen-8-yl)acetic acid (485 mg, 1.66 mmol) obtained in [Step-3] of the present example was used instead of ethyl 2-(4-methoxy-9-methyl-7-oxo-1,7-dihydro-2*H*-furo[3,2-t]chromen-8-yl)acetic acid in step 7) of Example 1.

¹H-NMR (300 MHz, CDCl₃): *δ* 6.53 (s, 1H), 6.09 (s, 2H), 3.96 (s, 3H), 3.76-3.65 (m, 10H), 2.56 (s, 3H).

### [Step 5] Preparation of 4-hydroxy-9-methyl-8-(2-morpholino-2-oxoethyl)-7H-[1,3]dioxolo[4,5-f]chromen-7-one

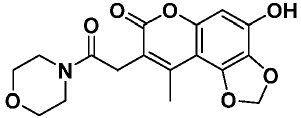

75 mg (26%, yield) of the target compound was obtained in the same manner as in step 8) of Example 1, except that 4-methoxy-9-methyl-8-(2-morpholino-2-oxoethyl)-7*H*-[1,3]dioxolo[4,5-*f*]chromen-7-one (300 mg, 0.83 mmol) prepared in step 4 of the present example was used instead of 4-hydroxy-9-methyl-8-(2-morpholino-2-oxoethyl)-1,2-dihydro-7H-furo[3,2-*f*]chloromen-7-one in step 8) of Example 1.

¹H-NMR (300 MHz, CD₃OD): *δ* 6.38 (s, 1H), 6.06 (s, 2H), 3.73-3.65 (m, 8H), 3.60-3.57 (m, 2H), 2.47 (s, 3H).

### [Step 6] Preparation of 4-hydroxy-9-methyl-8-(2-morpholino-2-oxoethyl)-7H-[1,3]dioxolo[4,5-f]chromene-5-carbaldehyde

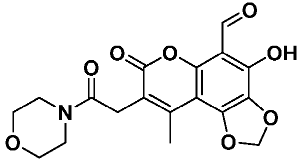

4 mg (5%, yield) of the target compound was obtained in the same manner as in step 9) of Example 1, except that 4-hydroxy-9-methyl-8-(2-morpholino-2-oxoethyl)-7H-[1,3]dioxolo[4,5-*f*]chromen-7-one (75 mg, 0.21 mmol) prepared in step 5 of the present example was used instead of 4-hydroxy-9-methyl-8-(2-morpholino-2-oxoethyl)-1,2-dihydro-7H-furo[3,2-*f*]chloromen-7-one in step 9) of Example 1.

¹H-NMR (300 MHz, CD₃OD): *δ* 10.35 (s, 1H), 6.16 (s, 2H), 3.74-3.58 (m, 10H), 2.46 (s, 3H).

MS (ESI⁺, m/z): 376.2 [M+H]⁺

### Example 3: 6-hydroxy-1-methyl-2-(2-morpholino-2-oxoethyl)-3-oxo-3,8,9,10-tetrahydropyrano[3,2-f]chromene-5-carbaldehyde

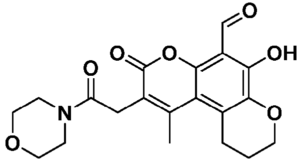

16 mg (6%, yield) of the target compound was obtained in the same manner as in Example 1, except that 3-chloropropan-1-ol (60 mL) was used instead of 2-chloroethanol in [Step-1] of Example 1.

¹H-NMR (300 MHz, CDCl₃): *δ* 12.55 (s, 1H), 10.59 (s, 1H), 4.35 (t, *J=* 5.0 Hz, 2H), 3.79-3.66 (m, 10H), 3.19 (t, *J*=6.3 Hz, 2H), 2.59 (s, 3H), 2.09-2.01 (m, 2H).

MS (ESI⁺, m/z): 388.2 [M+H]⁺

### Example 4: 6-hydroxy-1-methyl-2-(2-(4-methylpiperidin-1-yl)-2-oxoethyl)-3-oxo-3,8,9,10-tetrahydropyrano[3,2-f]chromene-5-carbaldehyde

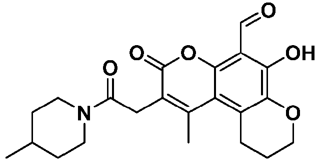

4 mg (2%, yield) of the target compound was obtained in the same manner as in Example 1, except that 3-chloropropan-1-ol (60 mL) was used instead of 2-chloroethanol in [Step-1] of Example 1, and 4-methylpiperidine (219 mg, 2.16 mmol) was used instead of morpholine in [Step-7] of Example 1.

¹H-NMR (300 MHz, CDCl₃): *δ* 10.56 (s, 1H), 4.56-4.51 (m, 1H), 4.33-4.30 (m, 1H), 4.10-4.04 (m, 1H), 3.80-3.66 (m, 5H), 3.17-3.13 (m, 2H), 2.65-2.57 (m, 1H), 2.56 (s, 3H), 2.05-2.00 (m, 2H), 1.98-1.66 (m, 2H), 1.23-1.09 (m, 2H), 0.98-0.96 (m, 3H)

MS (ESI⁺, m/z): 400.2 [M+H]⁺

### Example 5: 6-hydroxy-1-methyl-3-oxo-2-(2-oxo-2-(pyrrolidin-1-yl)ethyl)-3,8,9,10-tetrahydropyrano[3,2-f]chromene-5-carbaldehyde

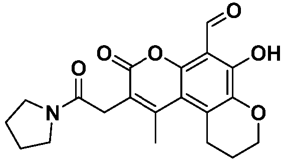

76 mg (47% yield) of the target compound was obtained in the same manner as in Example 1, except that 3-chloropropan-1-ol (60 mL) was used instead of 2-chloroethanol in [Step-1] of Example 1, and pyrrolidine (44 mg, 0.62 mmol) was used instead of morpholine in [Step-7] of Example 1.

¹H-NMR (300 MHz, CDCl₃): *δ* 12.53 (s, 1H), 10.59 (s, 1H), 4.34 (t, *J=* 5.0 Hz, 2H), 3.71-3.66 (m, 4H), 3.53- 3.48 (m, 2H), 3.19 (t, *J*= 6.3 Hz, 2H), 2.59 (s, 3H), 2.07-1.89 (m, 6H).

MS (ESI⁺, m/z): 372.2 [M+H]⁺

### Example 6: 6-hydroxy-1-methyl-2-(2-(4-methylpiperazin-1-yl)-2-oxoethyl)-3-oxo-3,8,9,10-tetrahydropyrano[3,2-f]chromene-5-carbaldehyde

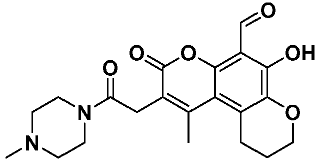

20 mg of the target compound (21%, yield) was obtained in the same manner as in Example 1, except that 3-chloropropan-1-ol (60 mL) was used instead of 2-chloroethanol in [Step-1] of Example 1, and 1-methylpiperazine (40 mg, 0.40 mmol) was used instead of morpholine in [Step-7] of Example 1.

¹H-NMR (300 MHz, CDCl₃): *δ* 12.55 (s, 1H), 10.59 (s, 1H), 4.34 (t, *J*= 5.1 Hz, 2H), 3.82-3.71 (m, 8H), 3.18 (t, *J*= 6.3 Hz, 2H), 2.64 (s, 3H), 2.57-2.42 (m, 2H), 2.44 (s, 3H), 2.08-1.98 (m, 2H).

MS (ESI⁺, m/z): 401.2 [M+H]⁺

### Example 7: 6-hydroxy-2-(2-(4-isopropylpiperazin-1-yl)-2-oxoethyl)-1-methyl-3-oxo-3,8,9,10-tetrahydropyrano[3,2-f]chromene-5-carbaldehyde

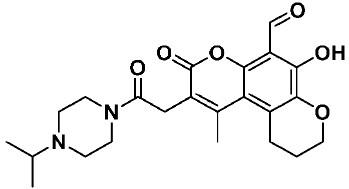

2 mg (1%, yield) of the target compound was obtained in the same manner as in Example 1, except that 3-chloropropan-1-ol (60 mL) was used instead of 2-chloroethanol in [Step-1] of Example 1, and 1-isopropylpiperazine (257 mg, 1.97 mmol) was used instead of morpholine in [Step-7] of Example 1.

¹H-NMR (300 MHz, CDCl₃): *δ* 10.38 (s, 1H), 4.24-4.19 (m, 2H), 3.75-3.72 (m, 2H), 3.66-3.35 (m, 4H), 3.34-3.06 (m, 2H), 2.76-2.70 (m, 1H), 2.65-2.53 (m, 4H), 2.45 (s, 3H), 1.97-1.89 (m, 2H), 1.10-1.08 (m, 6H).

MS (ESI⁺, m/z): 429.2 [M+H]⁺

### Example 8: N-ethyl-2-(5-formyl-4-hydroxy-9-methyl-7-oxo-1,7-dihydro-2H-furo[3,2-f]chromen-8-yl)acetamide

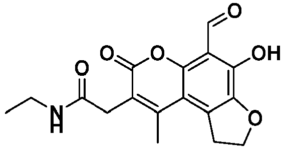

5 mg (10%, yield) of the target compound was obtained in the same manner as in Example 1, except that 2.0M methylamine tetrahydrofuran solution (39 mg, 0.86 mmol) was used instead of morpholine in [Step-7] of Example 1.

¹H-NMR (300 MHz, CDCl₃): *δ* 10.60 (s, 1H), 6.25 (brs, 1H), 4.79-4.72 (m, 2H), 3.85-3.68 (m, 2H), 3.58 (s, 2H), 3.32-3.22 (m, 2H), 2.67 (s, 3H), 1.18-1.12 (m, 3H).

MS (ESI⁺, m/z): 332.1 [M+H]⁺

### Example 9: N,N-diethyl-2-(5-formyl-4-hydroxy-9-methyl-7-oxo-1,7-dihydro-2H-furo[3,2-f]chromen-8-yl)acetamide

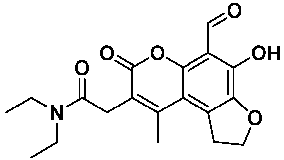

18 mg (25%, yield) of the target compound was obtained in the same manner as in Example 1, except that diethylamine (126 mg, 1.72 mmol) was used instead of morpholine in [Step-7] of Example 1.

¹H-NMR (300 MHz, CDCl₃): *δ*12.04 (brs, 1H), 10.59 (s, 1H), 4.78-4.71 (m, 2H), 3.75 (s, 2H), 3.74-3.67 (m, 2H), 3.59-3.38 (m, 4H), 2.51 (s, 3H), 1.37-1.13 (m, 6H).

MS (ESI⁺, m/z): 360.1 [M+H]⁺

### Example 10: 2-(2-(3,3-difluoropyrrolidin-1-yl)-2-oxoethyl)-6-hydroxy-1-methyl-3-oxo-3,8,9,10-tetrahydropyrano[3,2-f]chromene-5-carbaldehyde

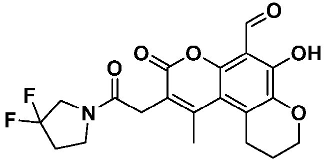

5 mg (19%, yield) of the target compound was obtained in the same manner as in Example 1, except that 3-chloropropan-1-ol (60 mL) was used instead of 2-chloroethanol in [Step-1] of Example 1, and 3,3-difluoropyrrolidine hydrochloride (193 mg, 1.31 mmol) was used instead of morpholine in [Step-7] of Example 1.

¹H-NMR (300 MHz, CDCl₃): *δ* 12.58 (brs, 1H), 10.59 (s, 1H), 4.37-4.33 (m, 2H), 4.14-3.64 (m, 6H), 3.22-3.16 (m, 2H), 2.61 (s, 3H), 2.58-2.02 (m, 4H).

MS (ESI⁺, m/z): 408.1 [M+H]⁺

### Example 11: 6-hydroxy-1-methyl-2-(2-(3-methylpyrrolidin-1-yl)-2-oxoethyl)-3-oxo-3,8,9,10-tetrahydropyrano[3,2-f]chromene-5-carbaldehyde

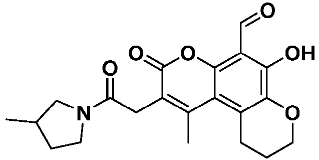

12 mg (19%, yield) of the target compound was obtained in the same manner as in Example 1, except that 3-chloropropan-1-ol (60 mL) was used instead of 2-chloroethanol in [Step-1] of Example 1, and 3-methylpyrrolidine hydrochloride (98 mg, 0.79 mmol) was used instead of morpholine in [Step-7] of Example 1.

¹H-NMR (300 MHz, CD₃OD): *δ* 10.46 (s, 1H), 4.84-4.24 (m, 2H), 3.89-2.57 (m, 4H), 3.30- 3.17 (m, 4H), 2.51(s, 3H), 2.48-2.01 (m, 4H), 1.99-1.51 (m, 1H), 1.15-0.91 (m, 3H).

MS (ESI⁺, m/z): 386.2 [M+H]⁺

### Example 12: 2-(2-(3,3-dimethylpyrrolidin-1-yl)-2-oxoethyl)-6-hydroxy-1-methyl-3-oxo-3,8,9,10-tetrahydropyrano[3,2-f]chromene-5-carbaldehyde

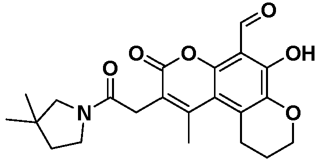

13 mg (8%, yield) of the target compound was obtained in the same manner as in Example 1, except that 3-chloropropan-1-ol (60 mL) was used instead of 2-chloroethanol in [Step-1] of Example 1, and 3,3-dimethylpyrrolidine (206 mg, 1.97 mmol) was used instead of morpholine in [Step-7] of Example 1.

¹H-NMR (300 MHz, CDCl₃): *δ* 12.50 (brs, 1H), 10.59 (s, 1H), 4.37-4.32 (m, 2H), 3.82-3.55 (m, 4H), 3.44-3.16 (m, 4H), 2.60 (s, 3H), 1.86-1.60 (m, 4H), 1.28 (s, 3H), 1.18 (s, 3H).

MS (ESI⁺, m/z): 400.2 [M+H]⁺

### Example 13: 2-(2-(azetidin-1-yl)-2-oxoethyl)-6-hydroxy-1-methyl-3-oxo-3,8,9,10-tetrahydropyrano[3,2-f]chromene-5-carbaldehyde

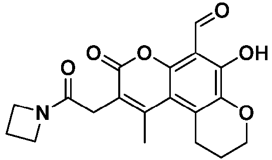

2 mg (3%, yield) of the target compound was obtained in the same manner as in Example 1, except that 3-chloropropan-1-ol (60 mL) was used instead of 2-chloroethanol in [Step-1] of Example 1, and azetidine (79 mg, 1.31 mmol) was used instead of morpholine in [Step-7] of Example 1.

¹H-NMR (300 MHz, CD₃OD): *δ* 10.47 (s, 1H), 4.41-4.36 (m, 2H), 4.28-4.22 (m, 2H), 4.06-4.01 (m, 2H), 3.52 (s, 2H), 3.21-3.14 (m, 2H), 2.55 (s, 3H), 2.40-2.29 (m, 2H), 2.00-1.97 (m, 2H).

MS (ESI⁺, m/z): 358.1 [M+H]⁺

### Example 14: 6-hydroxy-2-(2-(3-methoxypyrrolidin-1-yl)-2-oxoethyl)-1-methyl-3-oxo-3,8,9,10-tetrahydropyrano[3,2-f]chromene-5-carbaldehyde

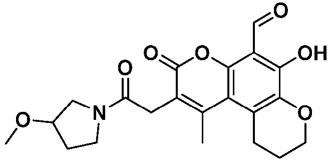

3 mg of the target compound (3%, yield) was obtained in the same manner as in Example 1, except that 3-chloropropan-1-ol (60 mL) was used instead of 2-chloroethanol in [Step-1] of Example 1, and 3-methoxypyrrolidine (98 mg, 0.69 mmol) was used instead of morpholine in [Step-7] of Example 1.

¹H-NMR (300 MHz, CD₃OD): *δ* 10.49 (s, 1H), 4.29-4.25 (m, 2H), 4.14-4.02 (m, 1H), 3.83-3.60 (m, 5H), 3.57-3.54 (m, 1H), 3.47 (s, 3H), 3.34-3.21 (m, 2H), 2.54 (s, 3H), 2.24-2.11 (m, 4H).

MS (ESI⁺, m/z): 402.2 [M+H]⁺

### Example 15: 6-hydroxy-2-(2-(3-methoxyazetidin-1-yl)-2-oxoethyl)-1-methyl-3-oxo-3,8,9,10-tetrahydropyrano[3,2-f]chromene-5-carbaldehyde

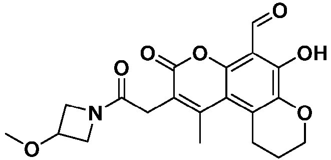

3 mg (10%, yield) of the target compound was obtained in the same manner as in Example 1, except that 3-chloropropan-1-ol (60 mL) was used instead of 2-chloroethanol in [Step-1] of Example 1, and 3-methoxyazetidine hydrochloride (90 mg, 0.69 mmol) was used instead of morpholine in [Step-7] of Example 1.

¹H-NMR (300 MHz, CD₃OD): *δ* 10.49 (s, 1H), 4.57-4.52 (m, 1H), 4.29-4.15 (m, 5H), 3.85-3.80 (m, 1H), 3.57 (s, 2H), 3.31 (s, 3H), 3.26-3.19 (m, 2H), 2.56 (s, 3H), 2.04-1.95 (m, 2H).

MS (ESI⁺, m/z): 388.1 [M+H]⁺

### Example 16: 2-(2-(3-fluoropyrrolidin-1-yl)-2-oxoethyl)-6-hydroxy-1-methyl-3-oxo-3,8,9,10-tetrahydropyrano[3,2-f]chromene-5-carbaldehyde

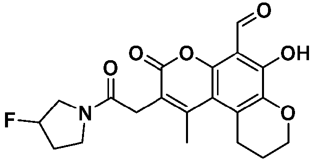

4 mg (2%, yield) of the target compound was obtained in the same manner as in Example 1, except that 3-chloropropan-1-ol (60 mL) was used instead of 2-chloroethanol in [Step-1] of Example 1, and 3-fluoropyrrolidine hydrochloride (116 mg, 0.90 mmol) was used instead of morpholine in [Step-7] of Example 1.

¹H-NMR (300 MHz, CD₃OD): *δ* 10.48 (s, 1H), 5.47-5.21 (m, 2H), 4.28-4.22 (m, 2H), 4.01-3.89 (m, 2H), 3.85-3.66 (m, 4H), 3.64-3.42 (m, 1H), 3.23-3.19 (m, 2H), 2.53 (s, 3H), 2.00-1.98 (m, 2H).

MS (ESI⁺, m/z): 390.1 [M+H]⁺

### Example 17: N-ethyl-2-(5-formyl-6-hydroxy-1-methyl-3-oxo-3,8,9,10-tetrahydropyrano[3,2-f]chromen-2-yl)acetamide

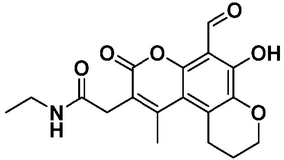

19 mg (7%, yield) of the target compound was obtained in the same manner as in Example 1, except that 3-chloropropan-1-ol (60 mL) was used instead of 2-chloroethanol in [Step-1] of Example 1, and 2.0 M ethylamine tetrahydrofuran solution (4.1 mL, 8.22 mmol) was used instead of morpholine in [Step-7] of Example 1.

¹H-NMR (300 MHz, CDCl₃): *δ* 12.56 (brs, 1H), 10.60 (s, 1H), 6.29 (s, 1H), 4.37-4.32 (m, 2H), 3.59 (s, 2H), 3.32-3.16 (m, 4H), 2.75 (s, 3H), 2.11-2.01 (m, 2H), 1.18-1.12 (m, 3H).

MS (ESI⁺, m/z): 346.1 [M+H]⁺

### Example 18: 1-ethyl-6-hydroxy-3-oxo-2-(2-oxo-2-(pyrrolidin-1-yl)ethyl)-3,8,9,10-tetrahydropyrano[3,2-f]chromene-5-carbaldehyde

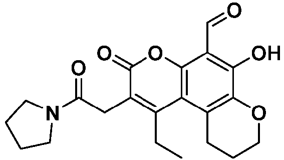

120 mg (49%, yield) of the target compound was obtained in the same manner as in Example 1, except that 3-chloropropan-1-ol (60 mL) was used instead of 2-chloroethanol in [Step-1] of Example 1, diethyl 2-propanoylbutanedioate (4.6 g, 19.99 mmol) was used instead of diethylacetyl succinate in [Step-5] of Example 1, and pyrrolidine (137 mg, 1.88 mmol) was used instead of morpholine in [Step-7] of Example 1.

¹H-NMR (300 MHz, CDCl₃): *δ* 12.56 (s, 1H), 10.56 (s, 1H), 4.33-4.29 (m, 2H), 3.73-3.69 (m, 4H), 3.55-3.50 (m, 2H), 3.20-3.16 (m, 2H), 3.04-2.97 (m, 2H), 2.09-2.03 (m, 4H), 1.97-1.24 (m, 2H), 1.24-1.19 (m, 3H).

MS (ESI⁺, m/z): 386.2 [M+H]⁺

### Example 19: 2-(2-(3-fluoroazetidin-1-yl)-2-oxoethyl)-6-hydroxy-1-methyl-3-oxo-3,8,9,10-tetrahydropyrano[3,2-f]chromene-5-carbaldehyde

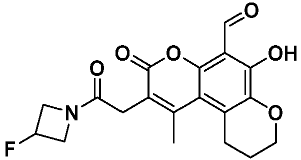

81 mg (19% yield) of the target compound was obtained in the same manner as in Example 1, except that 3-chloropropan-1-ol (60 mL) was used instead of 2-chloroethanol in [Step-1] of Example 1, and 3-fluoroazetidine hydrochloride (367 mg, 3.29 mmol) was used instead of morpholine in [Step-7] of Example 1.

¹H-NMR (300 MHz, CDCl₃): *δ* 12.55 (brs, 1H), 10.58 (s, 1H), 4.96-4.88 (m, 1H), 4.69-4.11 (m, 6H), 3.59-3.45 (m, 2H), 3.21-3.15 (m, 2H), 2.64 (s, 3H), 2.10-2.01 (m, 2H).

MS (ESI⁺, m/z): 376.1 [M+H]⁺

### Example 20: 6-hydroxy-1-methyl-3-oxo-2-(2-oxo-2-(5-azaspiro[2.4]heptan-5-yl)ethyl)-3,8,9,10-tetrahydropyrano[3,2-f]chromene-5-carbaldehyde

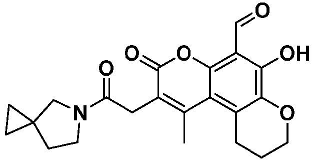

3 mg (1%, yield) of the target compound was obtained in the same manner as in Example 1, except that 3-chloropropan-1-ol (60 mL) was used instead of 2-chloroethanol in [Step-1] of Example 1, and 5-azaspiro[2.4]heptane (141 mg, 1.38 mmol) was used instead of morpholine in [Step-7] of Example 1.

¹H-NMR (300 MHz, CDCl₃): *δ* 12.54 (brs, 1H), 10.59 (s, 1H), 4.37-4.32 (m, 2H), 3.88-3.39 (m, 6H), 3.22-3.17 (m, 2H), 2.60 (s, 3H), 2.10-1.80 (m, 4H), 0.80-0.50 (m, 4H).

MS (ESI⁺, m/z): 398.2 [M+H]⁺

### Example 21: 6-hydroxy-1-methyl-3-oxo-2-(2-oxo-2-(piperidin-1-yl)ethyl)-3,8,9,10-tetrahydropyrano[3,2-f]chromene-5-carbaldehyde

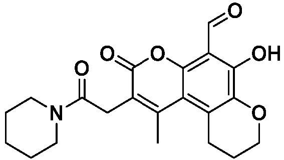

155 mg (39%, yield) of the target compound was obtained in the same manner as in Example 1, except that 3-chloropropan-1-ol (60 mL) was used instead of 2-chloroethanol in [Step-1] of Example 1, and piperidine (226 mg, 2.63 mmol) was used instead of morpholine in [Step-7] of Example 1.

¹H-NMR (300 MHz, CDCl₃): *δ* 12.52 (brs, 1H), 10.58 (s, 1H), 4.36-4.31 (m, 2H), 3.76 (s, 2H), 3.61-3.57 (m, 4H), 3.21-3.15 (m, 2H), 2.54 (s, 3H), 2.08-2.00 (m, 2H), 1.70-1.58 (m, 6H).

MS (ESI⁺, m/z): 386.2 [M+H]⁺

### Example 22: 2-(5-formyl-6-hydroxy-1-methyl-3-oxo-3,8,9,10-tetrahydropyrano[3,2-f]chromen-2-yl)acetamide

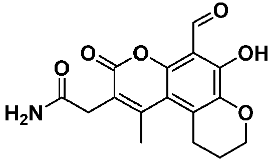

4 mg (2%, yield) of the target compound was obtained in the same manner as in Example 1, except that 3-chloropropan-1-ol (60 mL) was used instead of 2-chloroethanol in [Step-1] of Example 1, and ammonia water (0.5 mL) was used instead of morpholine in [Step-7] of Example 1.

¹H-NMR (300 MHz, DMSO-*d*₆): *δ* 10.20 (s, 1H), 4.07-4.02 (m, 2H), 3.44 (s, 2H), 2.90-2.86 (m, 2H), 2.33 (s, 3H), 1.85-1.76 (m, 2H).

MS (ESI⁺, m/z): 318.1 [M+H]⁺

### Example 23: 6-hydroxy-1-isopropyl-3-oxo-2-(2-oxo-2-(pyrrolidin-1-yl)ethyl)-3,8,9,10-tetrahydropyrano[3,2-f]chromene-5-carbaldehyde.

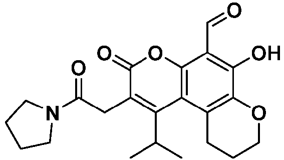

2 mg (4%, yield) of the target compound was obtained in the same manner as in Example 1, except that 3-chloropropan-1-ol (60 mL) was used instead of 2-chloroethanol in [Step-1] of Example 1, diethyl 2-(2-methylpropanoyl)butanedioate (2.76 g, 11.32 mmol) was used instead of diethylacetyl succinate in [Step-5] of Example 1, and pyrrolidine (46 mg, 0.63 mmol) was used instead of morpholine in [Step-7] of Example 1.

¹H-NMR (300 MHz, CDCl₃): *δ* 12.48 (s, 1H), 10.53 (s, 1H), 4.39-4.36 (m, 2H), 3.70-3.60 (m, 5H), 3.57-3.38 (m, 2H), 3.12-3.08 (m, 2H), 2.08-1.85 (m, 6H), 1.44 (s, 3H), 1.41 (s, 3H).

MS (ESI⁺, m/z): 400.2 [M+H]⁺

### Example 24: 2-(5-formyl-6-hydroxy-1-methyl-3-oxo-3,8,9,10-tetrahydropyrano[3,2-f]chromen-2-yl)-N-(2-methoxyethyl)acetamide

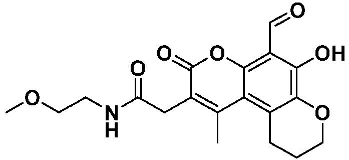

55 mg (21%, yield) of the target compound was obtained in the same manner as in Example 1, except that 3-chloropropan-1-ol (60 mL) was used instead of 2-chloroethanol in [Step-1] of Example 1, and 2-methoxyethylamine (504 mg, 6.57 mmol) was used instead of morpholine in [Step-7] of Example 1.

¹H-NMR (300 MHz, CDCl₃): *δ* 12.56 (brs, 1H), 10.60 (s, 1H), 6.52 (s, 1H), 4.37-4.32 (m, 2H), 3.62 (s, 2H), 3.50-3.40 (m, 4H), 3.38 (s, 3H), 3.21-3.16 (m, 2H), 2.72 (s, 3H), 2.10-2.01 (m, 2H).

MS (ESI⁺, m/z): 376.1 [M+H]⁺

### Example 25: N,N-diethyl-2-(5-formyl-6-hydroxy-1-methyl-3-oxo-3,8,9,10-tetrahydropyrano[3,2-f]chromen-2-yl)acetamide

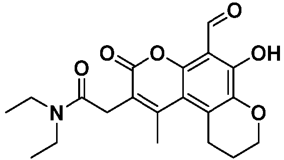

140 mg (19% yield) of the target compound was obtained in the same manner as in Example 1, except that 3-chloropropan-1-ol (60 mL) was used instead of 2-chloroethanol in [Step-1] of Example 1, and diethylamine (385 mg, 5.26 mmol) was used instead of morpholine in [Step-7] of Example 1.

¹H-NMR (300 MHz, CDCl₃): *δ* 12.53 (brs, 1H), 10.59 (s, 1H), 4.37-4.32 (m, 2H), 3.76 (s, 2H), 3.55-3.38 (m, 4H), 3.21-3.16 (m, 2H), 2.56 (s, 3H), 2.10-2.00 (m, 2H), 1.36-1.31 (m, 3H), 1.19-1.13 (m, 3H).

MS (ESI⁺, m/z): 374.2 [M+H]⁺

### Example 26: 2-(2-(4-acetylpiperazin-1-yl)-2-oxoethyl)-6-hydroxy-1-methyl-3-oxo-3,8,9,10-tetrahydropyrano[3,2-f]chromene-5-carbaldehyde

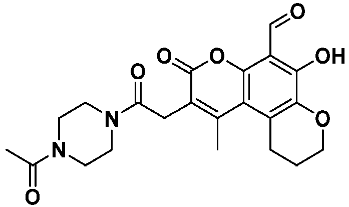

100 mg (28%, yield) of the target compound was obtained in the same manner as in Example 1, except that 3-chloropropan-1-ol (60 mL) was used instead of 2-chloroethanol in [Step-1] of Example 1, and 1-piperazine-1-ylethenone (258 mg, 1.97 mmol) was used instead of morpholine in [Step-7] of Example 1.

¹H-NMR (300 MHz, CDCl₃): *δ* 12.52 (s, 1H), 10.55 (s, 1H), 4.34-4.30 (m, 2H), 3.75-3.49 (m, 10H), 3.18-3.14 (m, 2H), 2.60-2.55 (m, 3H), 2.14 (s, 3H), 2.07-1.99 (m, 2H).

MS (ESI⁺, m/z): 429.2 [M+H]⁺

### Example 27: 2-(2-(2-oxa-5-azabicyclo[2.2.1]heptan-5-yl)-2-oxoethyl)-6-hydroxy-1-methyl-3-oxo-3,8,9,10-tetrahydropyrano[3,2-f]chromene-5-carbaldehyde

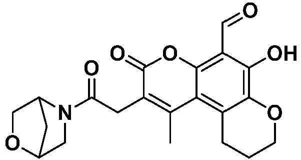

15 mg (7%, yield) of the target compound was obtained in the same manner as in Example 1, except that 3-chloropropan-1-ol (60 mL) was used instead of 2-chloroethanol in [Step-1] of Example 1, and 2-oxa-5-azabicyclo[2.2.1]heptane hydrochloride (273 mg, 1.97 mmol) was used instead of morpholine in [Step-7] of Example 1.

¹H-NMR (300 MHz, CDCl₃): *δ* 12.55 (brs, 1H), 10.58 (s, 1H), 4.97-4.92 (m, 1H), 4.76-4.68 (m, 1H), 4.37-4.33 (m, 2H), 3.99-3.40 (m, 6H), 3.22-3.17 (m, 2H), 2.68-2.60 (m, 3H), 2.10-1.95 (m, 4H).

MS (ESI⁺, m/z): 400.1 [M+H]⁺

### Example 28: 6-hydroxy-1-methyl-3-oxo-2-(2-oxo-2-thiomorpholinoethyl)-3,8,9,10-tetrahydropyrano[3,2-f]chromene-5-carbaldehyde

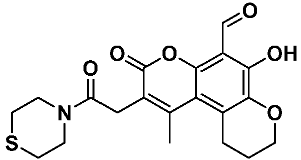

1.5 mg (1%, yield) of the target compound was obtained in the same manner as in Example 1, except that 3-chloropropan-1-ol (60 mL) was used instead of 2-chloroethanol in [Step-1] of Example 1, and thiomorpholine (109 mg, 1.03 mmol) was used instead of morpholine in [Step-7] of Example 1.

¹H-NMR (300 MHz, CDCl₃): *δ* 12.55 (brs, 1H), 10.59 (s, 1H), 4.37-4.32 (m, 2H), 3.99-3.90 (m, 4H), 3.75 (s, 2H), 3.21-3.16 (m, 2H), 2.79-2.64 (m, 4H), 2.57 (s, 3H), 2.10-2.00 (m, 2H).

MS (ESI⁺, m/z): 404.1 [M+H]⁺

### Example 29: 2-(5-formyl-6-hydroxy-1-methyl-3-oxo-3,8,9,10-tetrahydropyrano[3,2-f]chromen-2-yl)-N-(2-morpholinoethyl)acetamide

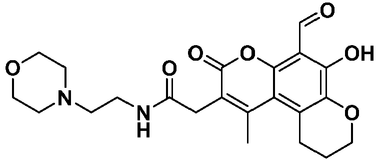

15 mg (7% yield) of the target compound was obtained in the same manner as in Example 1, except that 3-chloropropan-1-ol (60 mL) was used instead of 2-chloroethanol in [Step-1] of Example 1, and 4-(2-aminoethyl)morpholine (343 mg, 2.58 mmol) was used instead of morpholine in [Step-7] of Example 1.

¹H-NMR (300 MHz, CDCl₃): *δ* 12.55 (brs, 1H), 10.60 (s, 1H), 6.69 (s, 1H), 4.37-4.32 (m, 2H), 3.74-3.70 (m, 4H), 3.62 (s, 2H), 3.38-3.31 (m, 2H), 3.22-3.16 (m, 2H), 2.73 (s, 3H), 2.51-2.43 (m, 6H), 2.10-2.01 (m, 2H).

MS (ESI⁺, m/z): 431.2 [M+H]⁺

### Example 30: 6-hydroxy-1-methyl-3-oxo-2-(2-oxo-2-(thiazolidin-3-yl)ethyl)-3,8,9,10-tetrahydropyrano[3,2-f]chromene-5-carbaldehyde

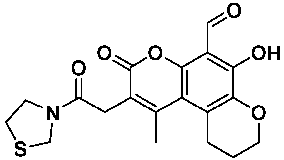

11 mg (4%, yield) of the target compound was obtained in the same manner as in Example 1, except that 3-chloropropan-1-ol (60 mL) was used instead of 2-chloroethanol in [Step-1] of Example 1, and thiazolidine (125 mg, 1.38 mmol) was used instead of morpholine in [Step-7] of Example 1.

¹H-NMR (300 MHz, CDCl₃): *δ* 12.55 (s, 1H), 10.57 (s, 1H), 4.34-4.31 (m, 2H), 3.60 (s, 2H), 3.46-3.40(m, 2H), 3.21-3.14 (m, 2H), 2.70 (s, 3H), 2.66-2.61 (m, 2H), 2.21 (s, 2H), 2.05-2.02 (m, 2H).

MS (ESI⁺, m/z): 390.2 [M+H]⁺

### Example 31: 6-hydroxy-1-methyl-3-oxo-2-(2-oxo-2-(piperazin-1-yl)ethyl)-3,8,9,10-tetrahydropyrano[3,2-f]chromene-5-carbaldehyde

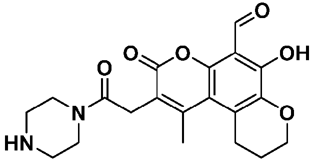

2 mg (7%, yield) of the target compound was obtained in the same manner as in Example 1, except that 3-chloropropan-1-ol (60 mL) was used instead of 2-chloroethanol in [Step-1] of Example 1, and piperazine (55 mg, 0.63 mmol) was used instead of morpholine in [Step-7] of Example 1.

¹H-NMR (300 MHz, CD₃OD): *δ* 10.51 (s, 1H), 4.29-4.26 (m, 2H), 3.81 (s, 2H), 3.76-3.67 (m, 6H), 3.62-3.59 (m, 2H), 3.25-3.21 (m, 2H), 2.52 (s, 3H), 2.05-1.97 (m, 2H).

MS (ESI⁺, m/z): 387.2 [M+H]⁺

According to an embodiment of the present disclosure, the compound of Formula 1 may be a compound selected from the group consisting of the compounds listed in Table 1 below.

**[Table 1]**

| Compound Number | Structure | Name | MS[M+H]⁺ |
|---|---|---|---|
| 1 | 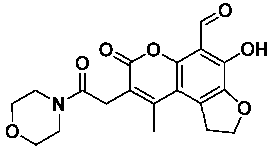 | 4-hydroxy-9-methyl-8-(2-morpholino-2-oxoethyl)-7-oxo-1,7-dihydro-*2H*-furo[3,2-*f*]chromene-5-carbaldehyde | 374.2 |
| 2 | 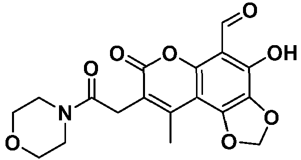 | 4-hydroxy-9-methyl-8-(2-morpholino-2-oxoethyl)-7-oxo-7*H*-[1,3]dioxolo[4,5-*f*]chromene-5-carbaldehyde | 376.2 |
| 3 | 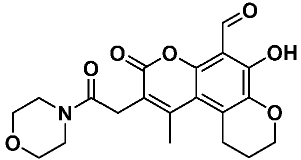 | 6-hydroxy-1-methyl-2-(2-morpholino-2-oxoethyl)-3-oxo-3,8,9,10-tetrahydropyrano[3,2-*f*]chromene-5-carbaldehyde | 388.2 |
| 4 | 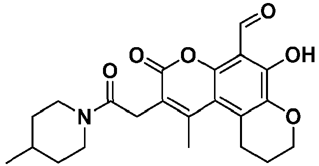 | 6-hydroxy-1-methyl-2-(2-(4-methylpiperidin-1-yl)-2-oxoethyl)-3-oxo-3,8,9,10-tetrahydropyrano[3,2-*f*]chromene-5-carbaldehyde | 400.2 |
| 5 | 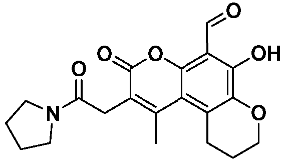 | 6-hydroxy-1-methyl-3-oxo-2-(2-oxo-2-(pyrrolidin-1-yl)ethyl)-3,8,9,10-tetrahydropyrano[3,2-flchromene-5-carbaldehyde | 372.2 |
| 6 | 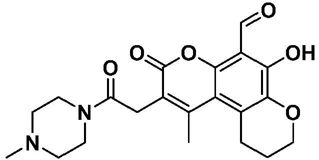 | 6-hydroxy-1-methyl-2-(2-(4-methylpiperazin-1-yl)-2-oxoethyl)-3-oxo-3,8,9,10-tetrahydropyrano[3,2-*f*]chromene-5-carbaldehyde | 401.2 |
| 7 | 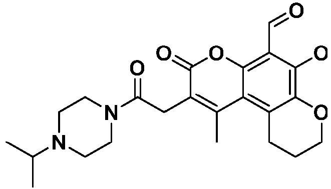 | 6-hydroxy-2-(2-(4-isopropylpiperazin-1-yl)-2-oxoethyl)-1-methyl-3-oxo-3,8,9,10-tetrahydropyrano[3,2-*f*]chromene-5-carbaldehyde | 429.2 |
| 8 | 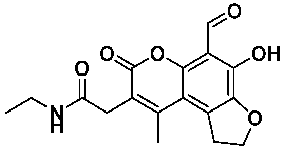 | *N*-ethyl-2-(5-formyl-4-hydroxy-9-methyl-7-oxo-1,7-dihydro-2*H*-furo[3,2-*f*]chromen-8-yl)acetamide | 332.1 |
| 9 | 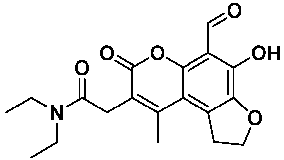 | *N*,*N*-diethyl-2-(5-formyl-4-hydroxy-9-methyl-7-oxo-1,7-dihydro-2*H*-furo[3,2-*f*]chromen-8-yl)acetamide | 360.1 |
| 10 | 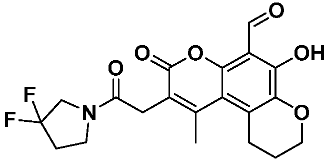 | 2-(2-(3,3-difluoropyrrolidin-1-yl)-2-oxoethyl)-6-hydroxy-1-methyl-3-oxo-3,8,9,10-tetrahydropyrano[3,2-*f*]chromene-5-carbaldehyde | 408.1 |
| 11 | 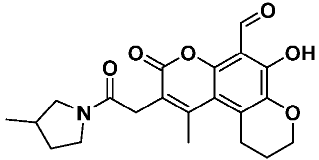 | 6-hydroxy-1-methyl-2-(2-(3-methylpyrrolidin-1-yl)-2-oxoethyl)-3-oxo-3,8,9,10-tetrahydropyrano[3,2-*f*]chromene-5-carbaldehyde | 386.2 |
| 12 | 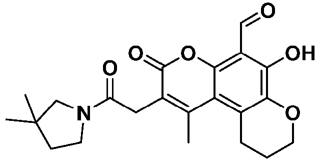 | 2-(2-(3,3-dimethylpyrrolidin-1-yl)-2-oxoethyl)-6-hydroxy-1-methyl-3-oxo-3,8,9,10-tetrahydropyrano[3,2-*f*]chromene-5-carbaldehyde | 400.2 |
| 13 | 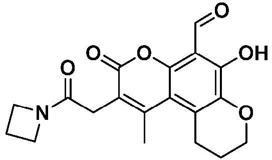 | 2-(2-(azetidin-1-yl)-2-oxoethyl)-6-hydroxy-1-methyl-3-oxo-3,8,9,10-tetrahydropyrano[3,2-*f*]chromene-5-carbaldehyde | 358.1 |
| 14 | 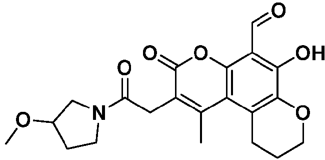 | 6-hydroxy-2-(2-(3-methoxypyrrolidin-1-yl)-2-oxoethyl)-1-methyl-3-oxo-3,8,9,10-tetrahydropyrano[3,2-*f*]chromene-5-carbaldehyde | 402.2 |
| 15 | 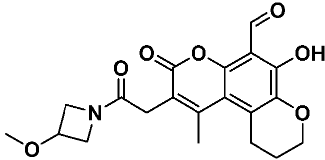 | 6-hydroxy-2-(2-(3-methoxyazetidin-1-yl)-2-oxoethyl)-1-methyl-3-oxo-3,8,9,10-tetrahydropyrano[3,2-*f*]chromene-5-carbaldehyde | 388.1 |
| 16 | 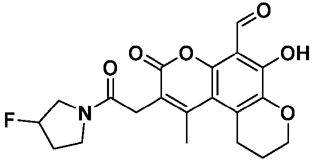 | 2-(2-(3-fluoropyrrolidin-1-yl)-2-oxoethyl)-6-hydroxy-1-methyl-3-oxo-3,8,9,10-tetrahydropyrano[3,2-*f*]chromene-5-carbaldehyde | 390.1 |
| 17 | 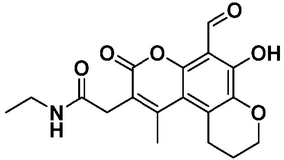 | *N*-ethyl-2-(5-formyl-6-hydroxy-1-methyl-3-oxo-3,8,9,10-tetrahydropyrano[3,2-*f*]chromen-2-yl)acetamide | 346.1 |
| 18 | 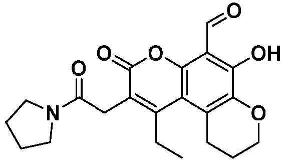 | 1-ethyl-6-hydroxy-3-oxo-2-(2-oxo-2-(pyrrolidin-1-yl)ethyl)-3,8,9,10-tetrahydropyrano[3,2-flchromene-5-carbaldehyde | 386.2 |
| 19 | 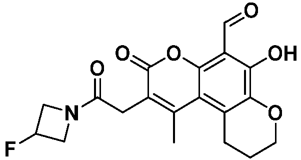 | 2-(2-(3-fluoroazetidin-1-yl)-2-oxoethyl)-6-hydroxy-1-methyl-3-oxo-3,8,9,10-tetrahydropyrano[3,2-*f*]chromene-5-carbaldehyde | 376.1 |
| 20 | 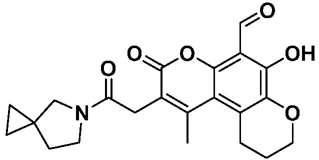 | 6-hydroxy-1-methyl-3-oxo-2-(2-oxo-2-(5-azaspiro[2.4]heptan-5-yl)ethyl)-3,8,9,10-tetrahydropyrano[3,2-*f*]chromene-5-carbaldehyde | 398.2 |
| 21 | 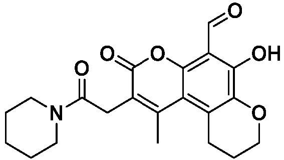 | 6-hydroxy-1-methyl-3-oxo-2-(2-oxo-2-(piperidin-1-yl)ethyl)-3,8,9,10-tetrahydropyrano[3,2-*f*]chromene-5-carbaldehyde | 386.2 |
| 22 | 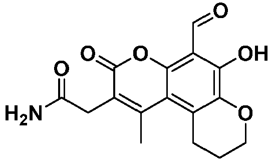 | 2-(5-formyl-6-hydroxy-1-methyl-3-oxo-3,8,9,10-tetrahydropyrano[3,2-*f*]chromen-2-yl)acetamide | 318.1 |
| 23 | 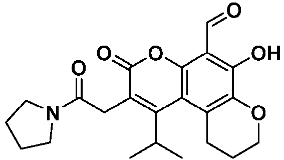 | 6-hydroxy-1-isopropyl-3-oxo-2-(2-oxo-2-(pyrrolidin-1-yl)ethyl)-3,8,9,10-tetrahydropyrano[3,2-*f*]chromene-5-carbaldehyde | 400.2 |
| 24 | 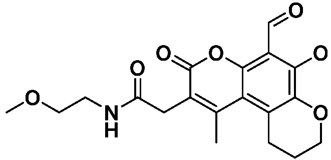 | 2-(5-formyl-6-hydroxy-1-methyl-3-oxo-3,8,9,10-tetrahydropyrano[3,2-*f*]chromen-2-yl)-*N*-(2-methoxyethyl)acetamide | 376.1 |
| 25 | 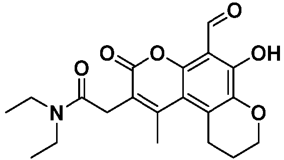 | *N,N*-diethyl-2-(5-formyl-6-hydroxy-1-methyl-3-oxo-3,8,9,10-tetrahydropyrano[3,2-*f*]chromen-2-yl)acetamide | 374.2 |
| 26 | 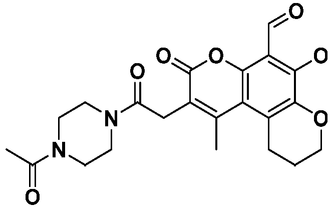 | 2-(2-(4-acetylpiperazin-1-yl)-2-oxoethyl)-6-hydroxy-1-methyl-3-oxo-3,8,9,10-tetrahydropyrano[3,2-*f*]chromene-5-carbaldehyde | 429.2 |
| 27 | 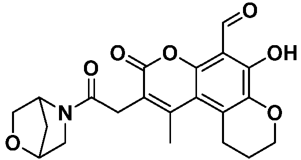 | 2-(2-(2-oxa-5-azabicyclo[2.2.1]heptan-5-yl)-2-oxoethyl)-6-hydroxy-1-methyl-3-oxo-3,8, 9,10-tetrahydropyrano[3,2-*f*]chromene-5-carbaldehyde | 400.1 |
| 28 | 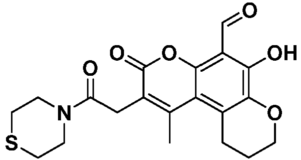 | 6-hydroxy-1-methyl-3-oxo-2-(2-oxo-2-thiomorpholinoethyl)-3,8,9,10-tetrahydropyrano[3,2-*f*]chromene-5-carbaldehyde | 404.1 |
| 29 | 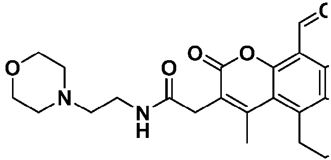 | 2-(5-formyl-6-hydroxy-1-methyl-3-oxo-3,8,9,10-tetrahydropyrano[3,2-*f*]chromen-2-yl)-*N*-(2-morpholinoethyl)acetamide | 431.2 |
| 30 | 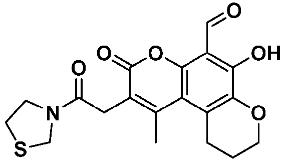 | 6-hydroxy-1-methyl-3-oxo-2-(2-oxo-2-(thiazolidin-3-yl)ethyl)-3,8,9,10-tetrahydropyrano[3,2-*f*]chromene-5-carbaldehyde | 390.1 |
| 31 | 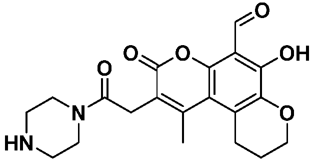 | 6-hydroxy-1-methyl-3-oxo-2-(2-oxo-2-(piperazin-1-yl)ethyl)-3,8,9,10-tetrahydropyrano[3,2-*f*]chromene-5-carbaldehyde | 387.2 |

### Experimental Example 1: Spliced XBP1 mRNA measurement test

With respect to these synthetic compounds, reverse transcription polymerase chain reaction (RT-PCR) experiments were conducted on MCF-7 cancer cells to confirm the effect thereof on the mRNA and protein amounts of spliced XBP1, which is the target of IRE endonuclease. A reverse transcription polymerase chain reaction is a test method in which RNA is extracted from cells, reverse transcriptase is used to make cDNA, and relative mRNA expression level is measured through a polymerase chain reaction.

Briefly, MCF-7 cells were seeded in a 12-well plate and cultured in an incubator including 5% CO₂ for one day. On the next day, compounds, which were 10-fold serially diluted from 10,000 nM, were pre-treated for 1 hour, and then, treated with 2 µg/mL tunicamycin for 6 or 24 hours to induce endoplasmic reticulum stress, followed by cell lysis to obtain RNA. RNA was subjected to the procedure of RNeasy Kits (Qiagen, 74106), and 5 µg of RNA sample was dissolved in a sample including reverse transcriptase and dNTP, and reverse transcription reaction was performed with a thermal cycler. The amount of mRNA of each of spliced XBP1 and total XBP1 was measured by a polymerase chain reaction through real time PCR by mixing each cDNA sample with a PCR reaction mixture including a primer pair capable of binding specifically to each target and SYBR green dye. The expression level of spliced XBP1 was corrected by the total expression level of XBP1, and the relative expression levels of spliced XBP1 between samples were compared and analyzed. The relative amount of spliced XBP1 RNA inside the compound-treated cells was normalized based on tunicamycin-treated samples (100%) and untreated samples (0%), and the percentage value of each sample was used to calculate the dose-response curve and IC₅₀ value according to the drug concentration. As a control material, the IRE1α RNase inhibitor MKC8866 (MedChemExpress, Cat #. HY-104040) was used. As a result of this, the inhibitory ability of the spliced XBP1 expression of the compound is shown in Table 2 below.

A indicates a case where the IC₅₀ value is less than 100 nM, B indicates a case where an IC₅₀ value is 100 nM or more and less than 200 nM, and C indicates a case where IC₅₀ value is 200 nM or more.

**[Table 2]**

| **Synthetic compound** | **XBP1 mRNA (IC₅₀, nM)** | **Synthetic compound** | **XBP1 mRNA (IC₅₀, nM)** |
|---|---|---|---|
| MKC8866 | A | Example 16 | C |
| Example 1 | A | Example 17 | A |
| Example 2 | B | Example 18 | C |
| Example 3 | A | Example 19 | B |
| Example 4 | C | Example 20 | B |
| Example 5 | A | Example 21 | C |
| Example 6 | A | Example 22 | C |
| Example 7 | B | Example 23 | B |
| Example 8 | A | Example 24 | A |
| Example 9 | A | Example 25 | A |
| Example 10 | A | Example 26 | C |
| Example 11 | A | Example 27 | A |
| Example 12 | B | Example 28 | B |
| Example 13 | B | Example 29 | C |
| Example 14 | B | Example 30 | A |
| Example 15 | B | Example 31 | C |

The present disclosure has been discussed in terms of embodiments. Those skilled in the art to which the present disclosure pertains would be able to understand that the present disclosure can be implemented in a modified form without departing from the essential characteristics of the present disclosure. Therefore, the disclosed embodiments are to be considered in an illustrative rather than a limiting sense. The scope of the present disclosure is shown in the claims rather than the foregoing description, and all differences within the equivalent scope will be construed as being included in the present disclosure.

## Claims

1. A compound selected from compounds of Formula 1, optical isomers, diastereomers, solvates and hydrates thereof, and pharmaceutically acceptable salts thereof: 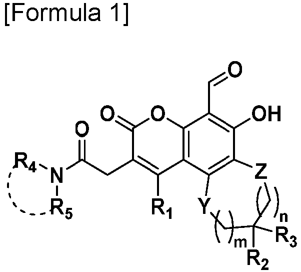 wherein, in Formula 1,
R₁ is hydrogen, halogen, hydroxy, cyano, C₁₋₆ alkyl, C₂₋₆ alkenyl, C₂₋₆ alkynyl, haloC₁₋₆ alkyl, -(CH₂)ₐ-C₁₋₆ alkoxy, -(CH₂)ₐ-NR₆R₇, substituted or unsubstituted -(CH₂)ₐ-C₃₋₆ cyclyl, substituted or unsubstituted -(CH₂)ₐ-C₆₋₁₀ aryl, or -(CH₂)ₐ-C₂₋₆ heterocyclyl;
R₂ and R₃ are each independently hydrogen, halogen, C₁₋₆ alkyl, haloC₁₋₆ alkyl, C₁₋₆ alkoxy, oxo(=O), or hydroxy;
a is an integer from 0 to 2;
n and m are each independently an integer from 0 to 2;
Z and Y are each independently -CH₂-, -O-, -S-, or -NR₈-;
a dotted line indicates the presence or absence of bonding;
R₄ and R₅ are each independently hydrogen, C₁₋₆ alkyl, C₂₋₆ alkenyl, C₂₋₆ alkynyl, C₁₋₆ alkoxy, C₁₋₆ alkoxyalkyl, C₃₋₁₀ cycloalkyl, C₆₋₁₀ aryl, C₄₋₁₀ heteroaryl, C₂₋₉ heterocycloalkyl, C₈₋₁₆ spirocycloalkyl, C₈₋₁₆ fused cycloalkyl, C₈₋₁₆ cross-linked cycloalkyl, C₆₋₁₄ heterospirocycloalkyl, C₆₋₁₄ fused heterocycloalkyl, or C₆₋₁₄ cross-linked heterocycloalkyl, each of which is unsubstituted or substituted with at least one substituent selected from halogen, nitro, oxo(=O), cyano, haloC₁₋₆ alkyl, C₁₋₆ alkyl, C₂₋₆ alkenyl, C₂₋₆ alkynyl, C₁₋₆ alkoxy, haloC₁₋₆ alkoxy, -S(O)-C₁₋₄ alkyl, -S(O)₂-C₁₋₄ alkyl, - C(O)-NR₉R₁₀, -C(O)OR₉, -OR₉, and -NR₉R₁₀;
alternatively, R₄ and R₅ are linked to each other to form, with an amide nitrogen of Formula 1, C₂₋₉ heterocyclyl, C₆₋₁₄ heterospirocyclyl, C₆₋₁₄ fused heterocyclyl, C₆₋₁₄ cross-linked heterocyclyl, or C₄₋₁₀ heteroaryl, each of which is unsubstituted or substituted with one or more substituents selected from halogen, nitro, oxo(=O), cyano, haloC₁₋₆ alkyl, C₁₋₆ alkyl, C₂₋₆ alkenyl, C₂₋₆ alkynyl, C₁₋₆ alkoxy, haloC₁₋₆ alkoxy, -C(O)R₁₁, -S(O)-C₁₋₄ alkyl, -S(O)₂-C₁₋₄ alkyl, -C(O)-NR₁₁R₁₂, -C(O)OR₁₁, -OR₁₁, and -NR₁₁R₁₂;
R₆, R₇, R₈, R₉, R₁₀, R₁₁, and R₁₂ are each independently hydrogen, C₁₋₆ alkyl, C₂₋₆ alkenyl, C₂₋₆ alkynyl, C₁₋₆ alkoxy, C₁₋₆ alkoxyalkyl, haloC₁₋₆ alkyl, C₆₋₁₀ aryl, C₃₋₁₀ cycloalkyl, C₄₋₁₀ heteroaryl, or C₂₋₉ heterocycloalkyl, alternatively, when R₁ is -(CH₂)ₐ-NR₆R₇, or when R₄ and R₅ are C(O)-NR₉R₁₀ or -NR₉R₁₀, a pair of R₆ and R₇, or a pair of R₉ and R₁₀, are linked to each other to form, with an amide nitrogen, substituted or unsubstituted C₂₋₉ heterocyclyl or substituted or unsubstituted C₄₋₁₀ heteroaryl.

2. The compound of claim 1, wherein
R₁ is C₁₋₆ alkyl, haloC₁₋₆ alkyl, -(CH₂)ₐ-C₁₋₆ alkoxy, -(CH₂)ₐ-NR₆R₇, or substituted or unsubstituted -(CH₂)ₐ-C₃₋₆ cyclyl.

3. The compound of claim 1, wherein
R₂ and R₃ are each independently hydrogen, halogen, or C₁₋₆ alkyl.

4. The compound of claim 1, wherein
Z and Y are each independently -CH₂- or -O-.

5. The compound of claim 1, wherein
R₄ and R₅ are each independently: hydrogen, C₁₋₆ alkyl, C₂₋₆ alkenyl, C₂₋₆ alkynyl, haloC₁₋₆ alkyl, C₁₋₆ alkoxyalkyl, C₃₋₁₀ cycloalkyl, or C₂₋₉ heterocycloalkyl; or C₁₋₆ alkyl substituted with C₂₋₉ heterocycloalkyl.

6. The compound of claim 1, wherein
R₄ and R₅ are linked to each other to form, with an amide nitrogen of Formula 1, C₂₋₉ heterocyclyl, C₆₋₁₄ heterospirocyclyl, C₆₋₁₄ cross-linked heterocyclyl, or C₄₋₁₀ heteroaryl, each being one selected from 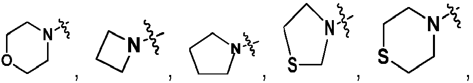 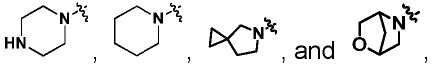 wherein the C₂₋₉ heterocyclyl, the C₆₋₁₄ heterospirocyclyl, the C₆₋₁₄ cross-linked heterocyclyl, or the C₄₋₁₀ heteroaryl is substituted with one or more substituents selected from hydrogen, halogen, nitro, cyano, haloC₁₋₆ alkyl, C₁₋₆alkyl, C₂₋₆ alkenyl, C₂₋₆ alkynyl, C₁₋₆ alkoxy, -C(O)R₁₁, haloC₁₋₆ alkoxy, and C₂₋₆ heterocyclyl.

7. The compound of claim 1, wherein
R₄ and R₅ are linked to each other to form, with an amide nitrogen of Formula 1, one selected from

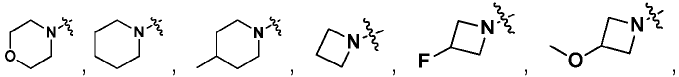

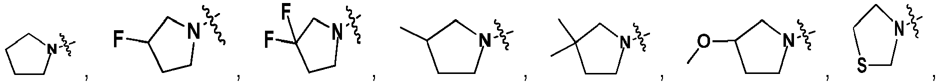

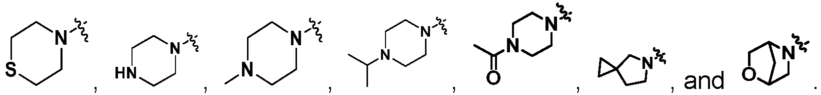

8. The compound of claim 1, wherein
the compound is a compound selected from compounds of Formula 2, optical isomers, diastereomers, solvates and hydrates thereof, and pharmaceutically acceptable salts thereof: 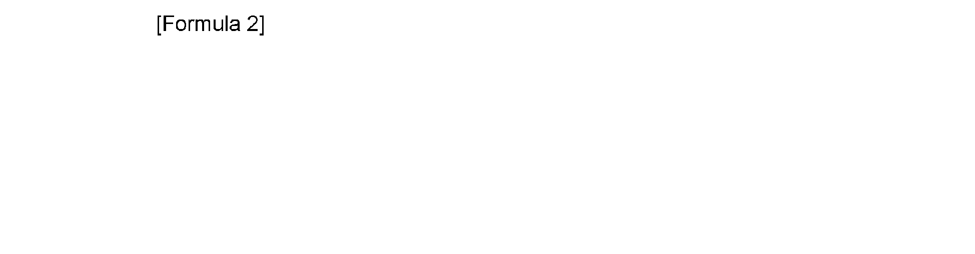 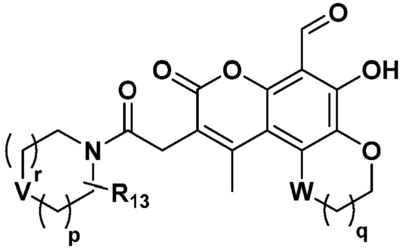 wherein, in Formula 2,
V and W are each independently -CH₂-, -S-, -O-, or -NR₁₄-;
p, q, and r are each independently an integer of 0 or 1;
R₁₃ is hydrogen, halogen, nitro, cyano, haloC₁₋₆ alkyl, C₁₋₆ alkyl, C₂₋₆ alkenyl, C₂₋₆ alkynyl, C₁₋₆ alkoxy, or haloC₁₋₆ alkoxy; and
each R₁₄ is independently hydrogen, C₁₋₆ alkyl, haloC₁₋₆ alkyl, or -C(O)(C₁₋₆ alkyl).

9. The compound of claim 1, wherein the compound is selected from the group consisting of the following compounds, optical isomers, diastereomers and solvates thereof, and pharmaceutically acceptable salts thereof:
1) 4-hydroxy-9-methyl-8-(2-morpholino-2-oxoethyl)-7-oxo-1,7-dihydro-2H-furo[3,2-*f*]chromene-5-carbaldehyde;
2) 4-hydroxy-9-methyl-8-(2-morpholino-2-oxoethyl)-7-oxo-7*H*-[1,3]dioxolo[4,5-flchromene-5-carbaldehyde;
3) 6-hydroxy-1-methyl-2-(2-morpholino-2-oxoethyl)-3-oxo-3,8,9,10-tetrahydropyrano[3,2-*f*]chromene-5-carbaldehyde;
4) 6-hydroxy-1-methyl-2-(2-(4-methylpiperidin-1-yl)-2-oxoethyl)-3-oxo-3,8,9,10-tetrahydropyrano[3,2-*f*]chromene-5-carbaldehyde;
5) 6-hydroxy-1-methyl-3-oxo-2-(2-oxo-2-(pyrrolidin-1-yl)ethyl)-3,8,9,10-tetrahydropyrano[3,2-*f*]chromene-5-carbaldehyde;
6) 6-hydroxy-1-methyl-2-(2-(4-methylpiperazin-1-yl)-2-oxoethyl)-3-oxo-3,8,9,10-tetrahydropyrano[3,2-*f*]chromene-5-carbaldehyde;
7) 6-hydroxy-2-(2-(4-isopropylpiperazin-1-yl)-2-oxoethyl)-1-methyl-3-oxo-3,8,9,10-tetrahydropyrano[3,2-*f*]chromene-5-carbaldehyde;
8) N-ethyl-2-(5-formyl-4-hydroxy-9-methyl-7-oxo-1,7-dihydro-2H-furo[3,2-*f*]chromen-8-yl)acetamide;
9) N,N-diethyl-2-(5-formyl-4-hydroxy-9-methyl-7-oxo-1,7-dihydro-2H-furo[3,2-*f*]chromen-8-yl)acetamide;
10) 2-(2-(3,3-difluoropyrrolidin-1-yl)-2-oxoethyl)-6-hydroxy-1-methyl-3-oxo-3,8,9,10-tetrahydropyrano[3,2-*f*]chromene-5-carbaldehyde;
11) 6-hydroxy-1-methyl-2-(2-(3-methylpyrrolidin-1-yl)-2-oxoethyl)-3-oxo-3,8,9,10-tetrahydropyrano[3,2-*f*]chromene-5-carbaldehyde;
12) 2-(2-(3,3-dimethylpyrrolidin-1-yl)-2-oxoethyl)-6-hydroxy-1-methyl-3-oxo-3,8,9,10-tetrahydropyrano[3,2-*f*]chromene-5-carbaldehyde;
13) 2-(2-(azetidin-1-yl)-2-oxoethyl)-6-hydroxy-1-methyl-3-oxo-3,8,9,10-tetrahydropyrano[3,2-*f*]chromene-5-carbaldehyde;
14) 6-hydroxy-2-(2-(3-methoxypyrrolidin-1-yl)-2-oxoethyl)-1-methyl-3-oxo-3,8,9,10-tetrahydropyrano[3,2-*f*]chromene-5-carbaldehyde;
15) 6-hydroxy-2-(2-(3-methoxyazetidin-1-yl)-2-oxoethyl)-1-methyl-3-oxo-3,8,9,10-tetrahydropyrano[3,2-*f*]chromene-5-carbaldehyde;
16) 2-(2-(3-fluoropyrrolidin-1-yl)-2-oxoethyl)-6-hydroxy-1-methyl-3-oxo-3,8,9,10-tetrahydropyrano[3,2-*f*]chromene-5-carbaldehyde;
17) N-ethyl-2-(5-formyl-6-hydroxy-1-methyl-3-oxo-3,8,9,10-tetrahydropyrano[3,2-*f*]chromen-2-yl)acetamide;
18) 1-ethyl-6-hydroxy-3-oxo-2-(2-oxo-2-(pyrrolidin-1-yl)ethyl)-3,8,9,10-tetrahydropyrano[3,2-*f*]chromene-5-carbaldehyde;
19) 2-(2-(3-fluoroazetidin-1-yl)-2-oxoethyl)-6-hydroxy-1-methyl-3-oxo-3,8,9,10-tetrahydropyrano[3,2-*f*]chromene-5-carbaldehyde;
20) 6-hydroxy-1-methyl-3-oxo-2-(2-oxo-2-(5-azaspiro[2.4]heptan-5-yl)ethyl)-3,8,9,10-tetrahydropyrano[3,2-*f*]chromene-5-carbaldehyde;
21) 6-hydroxy-1-methyl-3-oxo-2-(2-oxo-2-(piperidin-1-yl)ethyl)-3,8,9,10-tetrahydropyrano[3,2-*f*]chromene-5-carbaldehyde;
22) 2-(5-formyl-6-hydroxy-1-methyl-3-oxo-3,8,9,10-tetrahydropyrano[3,2-*f*]chromen-2-yl)acetamide;
23) 6-hydroxy-1-isopropyl-3-oxo-2-(2-oxo-2-(pyrrolidin-1-yl)ethyl)-3,8,9,10-tetrahydropyrano[3,2-*f*]chromene-5-carbaldehyde;
24) 2-(5-formyl-6-hydroxy-1-methyl-3-oxo-3,8,9,10-tetrahydropyrano[3,2-*f*]chromen-2-yl)-*N*-(2-methoxyethyl)acetamide;
25) N,N-diethyl-2-(5-formyl-6-hydroxy-1-methyl-3-oxo-3,8,9,10-tetrahydropyrano[3,2-*f*]chromen-2-yl)acetamide;
26) 2-(2-(4-acetylpiperazin-1-yl)-2-oxoethyl)-6-hydroxy-1-methyl-3-oxo-3,8,9,10-tetrahydropyrano[3,2-*f*]chromene-5-carbaldehyde;
27) 2-(2-(2-oxa-5-azabicyclo[2.2.1]heptan-5-yl)-2-oxoethyl)-6-hydroxy-1-methyl-3-oxo-3,8,9,10-tetrahydropyrano[3,2-*f*]chromene-5-carbaldehyde;
28) 6-hydroxy-1-methyl-3-oxo-2-(2-oxo-2-thiomorpholinoethyl)-3,8,9,10-tetrahydropyrano[3,2-*f*]chromene-5-carbaldehyde;
29) 2-(5-formyl-6-hydroxy-1-methyl-3-oxo-3,8,9,10-tetrahydropyrano[3,2-*f*]chromen-2-yl)-*N*-(2-morpholinoethyl)acetamide;
30) 6-hydroxy-1-methyl-3-oxo-2-(2-oxo-2-(thiazolidin-3-yl)ethyl)-3,8,9,10-tetrahydropyrano[3,2-*f*]chromene-5-carbaldehyde; and
31) 6-hydroxy-1-methyl-3-oxo-2-(2-oxo-2-(piperazin-1-yl)ethyl)-3,8,9,10-tetrahydropyrano[3,2-*f*]chromene-5-carbaldehyde.

10. A pharmaceutical composition for treating or preventing inositol requiring enzyme 1α (IRE1α)-associated diseases, including, as an active ingredient, a compound selected from the compound of any one of claims 1-9, optical isomers, diastereomers, solvates and hydrates thereof, and pharmaceutically acceptable salts thereof.

11. The pharmaceutical composition of claim 10, wherein the pharmaceutical composition exhibits IRE1α inhibitory activity.

12. The pharmaceutical composition of claim 10, wherein the pharmaceutical composition exhibits IRE1α inhibitory activity and is for treating cancers or tumors that are treatable by IRE1α inhibitory activity.
